# EUROPEAN PATENT SPECIFICATION

(11) **EP 1 314 029 B1**
(45) Date of publication and mention of the grant of the patent: **13.04.2005**
(21) Application number: 01963153.0
(22) Date of filing: 29.08.2001
(51) Int. Cl.: G01N 33/487, G01N 33/49

(54) **DEVICE FOR MEASURING ANALYTE CONCENTRATION IN A FLUID**
VORRICHTUNG ZUR KONZENTRATIONSMESSUNG FÜR EIN ANALYT IN EINER FLÜSSIGKEIT
DISPOSITIF D'ANALYSE

(30) Priority: 30.08.2000 GB 0021219
(43) Date of publication of application: 28.05.2003
(73) Proprietor: HYPOGUARD LIMITED, Woodbridge Suffolk IP12 1PE (GB)
(72) Inventor: MAISEY, Graham, Antony, Chatham, Kent ME54 6EJ (GB); AITKEN, James, Dartford, Kent DA1 4SN (GB); WOODHEAD, Andrew, James, London WC1H 9NX (GB); MAY, Stuart, Richard, Hammersmith, London W6 9EW (GB); PEARSON, Michael, West Molesey, Surrey KT9 1QZ (GB); BLACK, Murdo, M., Forfar, Angus, DD8 2AZ (GB); SMITH, George, Elcoate, Abbeydale, Gloucestershire GL4 9XW (GB)
(74) Representative: Gemmell, Peter Alan, Dr.
(86) International application number: PCT/GB2001/003858
(87) International publication number: WO 2002/018940

(56) References cited:
- EP-A- 0 732 590
- WO-A-94/10558
- DE-A- 19 639 226

## Description

### 1. Field of the Invention

The present invention relates to a test device for measuring the concentration of an analyte in a fluid sample, notably to a test device for analysing blood glucose or other analytes in bodily fluids.

### 2. Description of the Prior Art

Diabetics regularly need to test samples of their blood to determine the level of blood glucose. The results of such tests may be used to determine levels of medication needed to treat the diabetes at the time. In one known type of system, disposable sensors are used to test the blood. The sensors typically take the form of test strips which are provided with a reagent material that will react with blood glucose to produce an electrical signal. Conductive tracks on the test strip relay the electrical signal to a meter which displays the result. After a sample of blood has been applied to the test strip and the measurement has been taken, the test strip is disposed of. In order to couple the conductive tracks on a test strip with the meter, the test strip needs to be inserted into a sensor holder prior to the start of testing. The sensor holder has corresponding electrodes which are brought into electrical contact with the conductive tracks of the test strip. Test devices are known in which a plurality of test strip are provided on a cartridge disc. Each strip is housed in its own sensor slot, and means are provided to eject a test strip from its slot when required, and to automatically locate it in a sensor holder. Examples of test devices with test strip dispensers are described in US Patent No. 5,660,791, European Patent Application No. 0 732 590, and European Patent Application No. 0 738 666.

A problem with test strips is that they have only a limited shelf life, and exposure of test strips to the atmosphere reduces the shelf life further. Test strips open to the atmosphere will typically have a shelf life of about two to three months, whereas test strips which are sealed from the atmosphere will have a shelf life of about six to 12 months.

It has been proposed in WO 94/10558 to provide a stack of disposable test elements in a cylindrical housing, the stack being urged towards a test station to form a liquidproof seal. In DE 196 39 226 Al it is proposed to provide a test device with a cartridge that may have a plurality of chambers containing test strips, each of which chambers may be individually sealed to preserve the shelf life of the strips therein. A user removes the seal for each chamber when required, and a timing circuit may be activated either by the user or when the cartridge is pushed into the device. After a set time period has elapsed, an alarm or other indication reminds the user that the time period for using the strips has elapsed.

It is an object of the present invention to provide an improved test device.

### SUMMARY OF THE INVENTION

According to the present invention there is provided a test device for testing of analyte concentration in a fluid to be applied thereto, the device comprising:
a) a plurality of sensors arranged in a stack, each of said sensors carrying reagent means for producing an electrical signal in response to the concentration of analyte in an applied fluid, each of said sensors having a plurality of electrode tracks for transmitting said electrical signal;
b) a housing having an opening therein and containing the said stack of sensors;
c) electrical contacts mounted in relation to the housing for engaging with electrode tracks on a sensor at an engagement location;
c) a meter connected to the said electrical contacts, having electronics means for producing a signal output which is dependent on the electrical signal from a sensor when the sensor is engaged with the said contacts;
d) a transport member rotatably mounted in the opening of the housing, having an axis of rotation which spans the opening and having an outer surface which is provided with a recessed region adapted to receive a single sensor from the stack;
e) spring means within the housing which urge the stack of sensors towards the transport member and which urge a single sensor into the said recess when the recess is suitably aligned adjacent to the stack;
f) sealing means for making a moisture tight seal between the transport member and the stack when the transport member is in a specified rotational position; and
g) wherein rotation of the transport member with a sensor in the recessed region will transport the sensor to the engagement location or to a position where the sensor can be moved to the engagement location, whereby electrode tracks of the sensor can engage with the said electrical contacts.

With the transport member in a start or home position, the sealing means keeps moisture from sensors in the stack when the device is not being used. The device may be factory assembled under controlled temperature and humidity conditions, and kept sealed until a user is ready to use a sensor. In a preferred embodiment the sealing means also provide a moisture-proof seal when the sensor is in the engagement location, so that sensors in the stack are protected while an analyte measurement is being taken.

The invention will be described with reference to the testing of glucose concentrations in blood, but it will be understood that the invention is not limited to this embodiment and is of general applicability for testing analytes in bodily and other fluids.

The transport member may take the sensor to the engagement location by rotational motion alone. Additional pushing means may be provided to translate the test strip to the engagement location. Such pushing means may be provided by a captured pusher which engages with a track on the housing so as to move the test strip during rotation of the transport member. Alternatively, separately actuatable pushing means could be provided to push the sensor to the engagement location after rotation of the transport member to a suitable position. It will also be understood that other means could be provided for moving the test strip after it has been carried by the transport member, for example rotation or a combination of rotation and translation.

The electrical contacts may be brought automatically into contact with electrode tracks on the sensor in the engagement location, for example by means of spring biasing. Alternatively, the user could move the electrical contacts to achieve the necessary contact at the engagement location.

After a measurement has been taken, it would be possible to remove the used sensor from the engagement location by manual intervention from the user. However, to avoid the need for handling of the used sensor it is preferred that the transport member can be further rotated from the engagement location to an exit location at which the sensor can fall out or be pushed out by ejection means.

After completion of a test measurement, and ejection of the used sensor, the transport member is returned or advanced to the start position. This may be done manually by the user or by means of a motor. The transport member is preferably urged by a return spring to return to the start position.

For simplicity the transport member is preferably circular in cross section, for example a cylindrical feed barrel. However the transport member could have a sectional shape that is not circular, for example oval, square or triangular.

The sealing means may be provided on an external surface of the transport member, or separate sealing means may be provided that make a seal between the transport member and the housing or the stack of sensors. Where the seal is made with the housing, the opening in which the transport member is mounted must of course be the only opening to the inside of the housing. In a preferred embodiment the sealing means comprises a retractable shroud which surrounds the stack of sensors and which sealingly engages with the transport member when in an extended configuration. In another preferred embodiment, the sealing means is provided by a seal on a door which opens and closes the opening according to the rotational position of the transport member. The door may be provided with one or more teeth to restrain movement of the stack, and the transport member may be provided with one or more blades to take over the restraining function from the teeth when the door is open, prior to the recessed region of the transport member being brought into register with the stack. This embodiment has the advantage that the door may remain closed, and the seal intact, at all times except for the brief period when a sensor is being loaded and taken to the engagement location. The sealing means may be formed from any suitable material, for example natural or synthetic rubber.

The sensors may be conventional test strips of known construction. The invention will for convenience be described hereinafter with reference to test strips; however it is to be understood that other shapes of sensor could also be employed, for example square sensors or discs.

The invention provides a test device in which test strips are dispensed via a rotationally mounted transport member so that each test strip is conveniently brought to a location where it can engage with meter contacts and where it can receive a drop of fluid.

In a preferred embodiment, each test strip comprises a base member having a working area to which the fluid is to be applied, containing the reagent means, and a non-working area adjacent to the working area, wherein the total thickness of the test member in at least a portion of the non-working area is at least as great as the total thickness of the test member in the working area.

By making the non-working area at least as thick as the working area, scuffing or abrasion of the working area in a stack can be reduced. Moreover, if a compressive load is applied to a stack of the test members, this may be spread out over a greater area, thereby reducing the possibility of compressive damage to the working area.

In a preferred embodiment, at least a part of the non-working area is of greater total thickness than the thickness of the working area. This further reduces the likelihood of damage to the working area by scuffing or abrasion when in a stack. The difference in thickness is preferably from 1 to 20 *µ*m, notably from 5 to 10 *µ*m.

To build up the working area, a plurality of layers are sequentially applied to the base layer, for example by screen printing, typically with curing or drying steps between the application steps. The layers which are printed typically comprise electrode patterns, a reagent layer, and a mesh layer (for spreading out an applied fluid). As a result of the application of these layers, the working area of a conventional electrochemical test strip is typically about 100 *µ*m thicker than the non-working area, which contains the electrode tracks and, typically, a dielectric layer. A stack of 100 test strips will therefore be about 10 mm thicker in the working area than in the non-working area. In a test strip in accordance with the present invention, at least a part of the non-working area may be made thicker by any suitable means. Suitable means include, for example: a printed relief ink; an applied pad or tape; embossing of the base layer or an intermediate layer; or an extension of the mesh layer from the working area.

Further objects and advantages of the invention will be apparent as the description proceeds.

The term "spring means" is used herein for convenience.
It will be understood that this term includes conventional springs and other biasing or urging means which perform an equivalent function; for example elastic or pneumatic biasing means.

### DETAILED DESCRIPTION OF THE DRAWINGS

The invention will now be further described, by way of example, with reference to the following drawings in which:
Figure 1 shows perspective views of a test device in accordance with a first embodiment of the present invention;
Figure 2 shows horizontal sectional views through the device of Figure 1a, along the line C-C of Figure 4) with the sealing means extended and retracted;
Figures 3 and 4 show vertical sectional views along the lines A-A and B-B respectively of Figure 2;
Figures 5 and 6 show vertical sectional views corresponding to those of Figures 3 and 4 respectively, but with the transport member progressively rotated;
Figure 7 shows respective sectional views along the lines E-E, F-F and G-G of Figures 5 and 6;
Figure 8 shows perspective views of a test device in accordance with a second embodiment of the present invention;
Figure 9 is a top plan view of the test device of Figure 8a;
Figure 10 is a sectional view along the line A-A of Figure 9;
Figure 11 is a sectional view along the line B-B of Figure 10;
Figure 12 shows sectional views corresponding to that of Figure 10, with the transport member at different rotational positions;
Figure 13 shows top plan views similar to Figure 9, with a test strip in different positions during the course of a test measurement;
Figure 14 shows perspective views of a test device in accordance with a third embodiment of the present invention;
Figure 15 is a top plan view of the test device of Figure 14a;
Figure 16 is a sectional view along the line A-A of Figure 15;
Figure 17 is a sectional view along the line B-B of Figure 16;
Figure 18 shows sectional views corresponding to that of Figure 16, with the transport member at different rotational positions;
Figure 19 shows top plan views similar to Figure 15, with a test strip in different positions during the course of a test measurement;
Figure 20 is a top plan view of a test strip in a preferred embodiment of the invention;
Figures 21 to 24 are sectional views through part of a further alternative embodiment, showing the operation of a sliding stop member;
Figure 25 shows sectional views through a device in accordance with an embodiment of the invention, showing operation of a magazine ratchet mechanism;
Figure 26 shows sectional views along the line A-A in Figure 25;
Figures 27 to 39 are perspective views showing a test device in accordance with another embodiment of the invention and its assembly.

### DETAILED DESCRIPTION

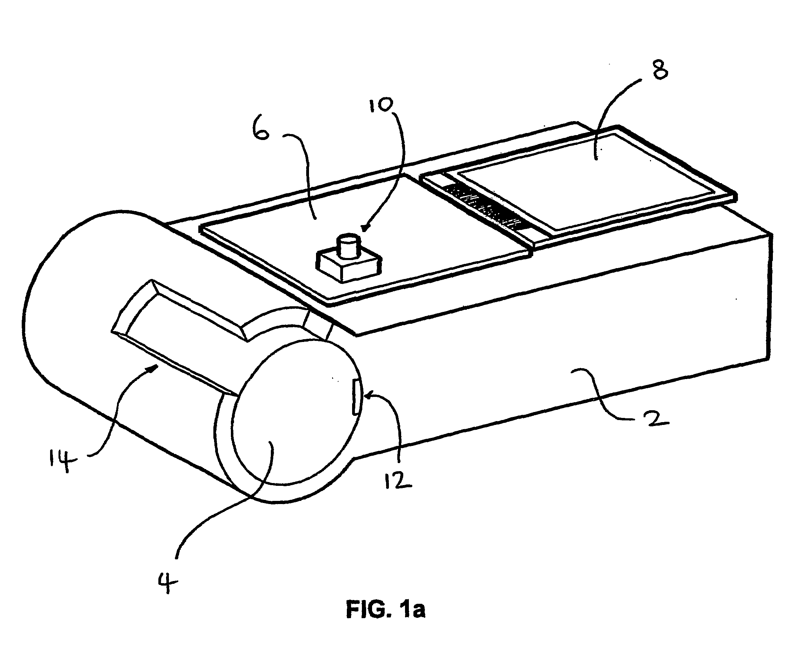

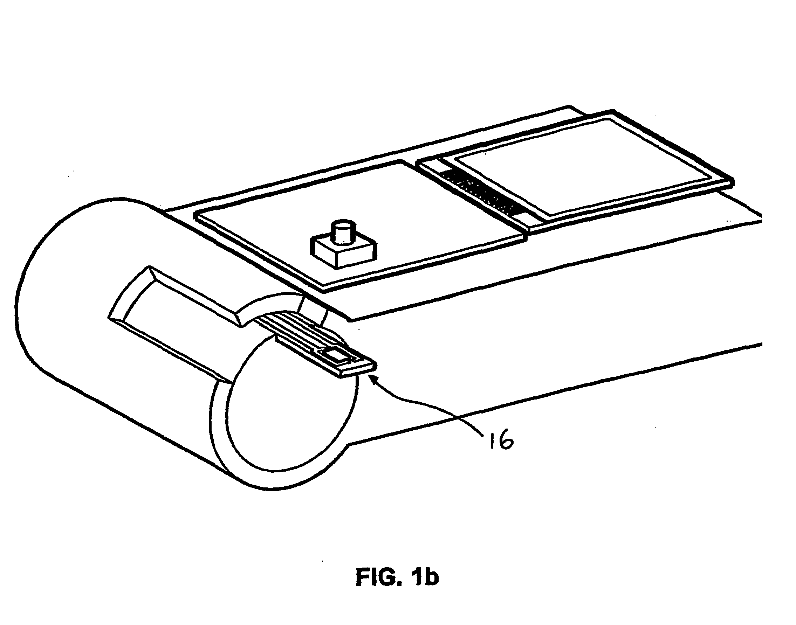

In the embodiment illustrated in Figures 1 to 7, a test device for measuring glucose concentration in blood comprises a housing 2 which houses a stack of test strips 16 in a magazine 18. A transport member 4 (in this example, a feed barrel which is circular in cross section) is rotatably mounted in an opening of the housing 2 and has an axis of rotation which spans the opening. As will be described below, the feed barrel 4 has a recessed portion 12 which receives and transports a single test strip 16 from the stack in the magazine from a start position (Figure 1a) to an engagement location (Figure 1b) where electrode tracks 50 on the test strip engage with electrical contacts mounted in the housing and connected to a meter. With the test strip 16 in the engagement, location a user can apply a drop of blood to the working area 42 of the test strip 16. The housing has mounted thereon a meter comprising a PCB 6 and display means 8 (in this example, an LCD) for displaying a readout of blood glucose concentration. Glucose concentration values from previous samples can be retained and displayed by operation of a memory button 10 on the PCB. Further rotation of the feed barrel 4 brings the used test strip 16 to an opening 14 in the housing 4 through which the test strip can be ejected or removed.

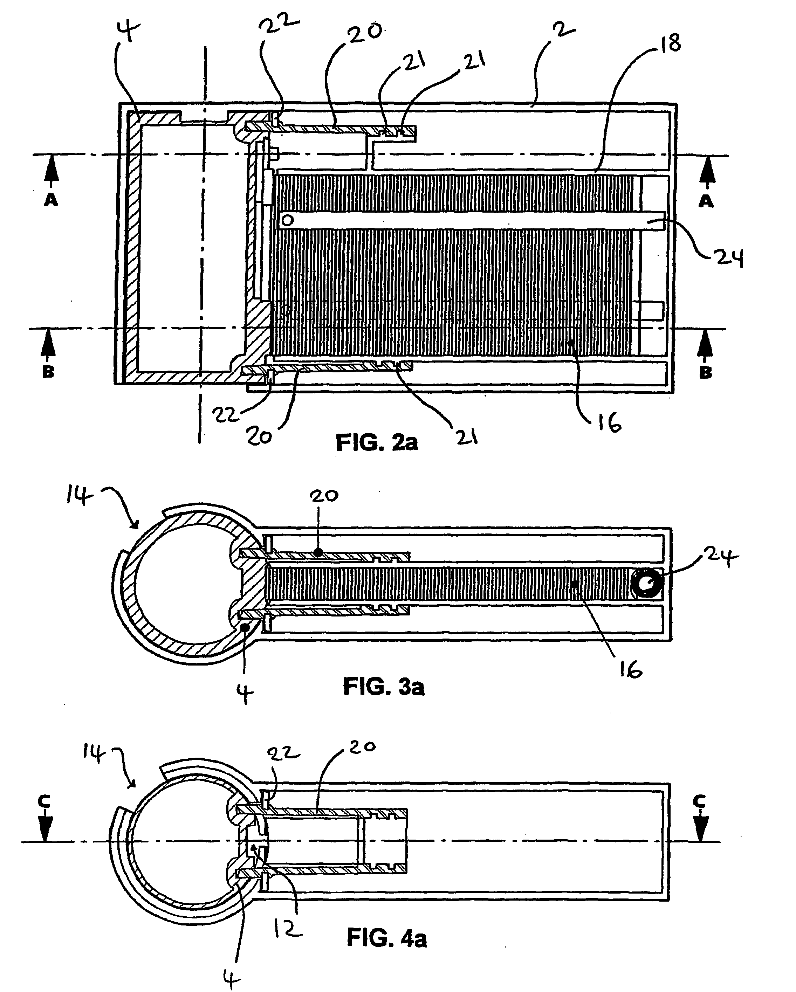

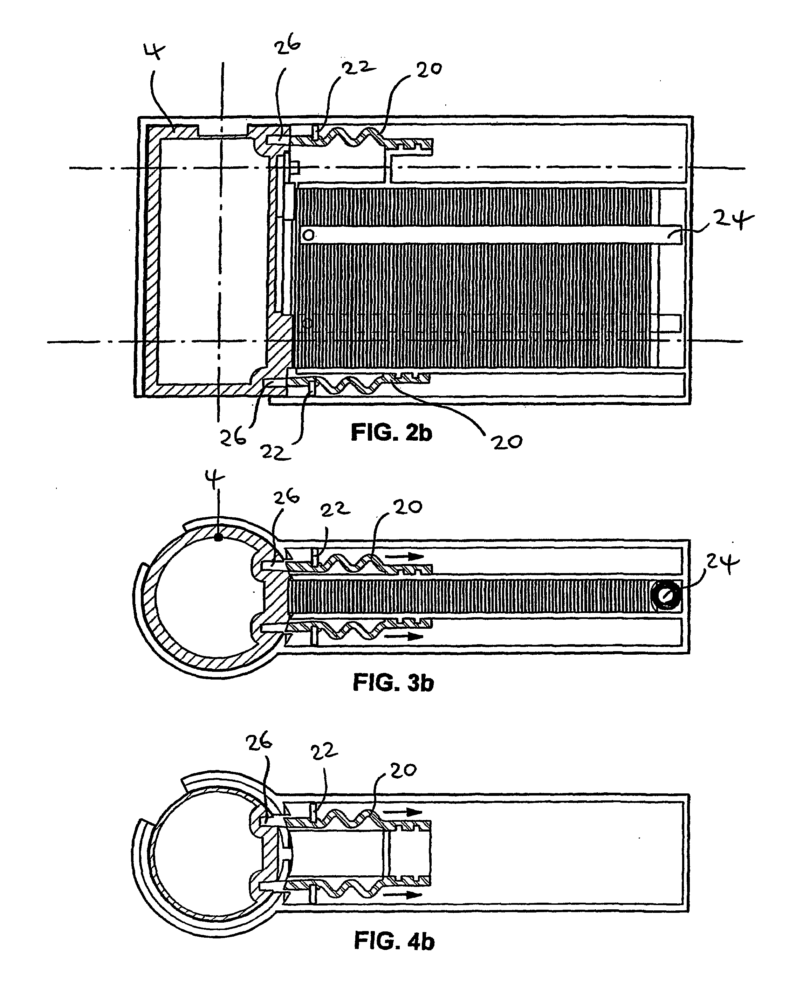

As shown in Figures 2 to 6, test strips 16 in the magazine 18 are protected by a moisture-proof retractable sealing member 20, which is tightly fitted around ribs 21 on the magazine 18. The free end of the retractable sealing member 20 is located in a groove 26 which surrounds the recess 12 where the test strip 16 is located in the start position. The retractable sealing member 20 is engaged by a movable support collar 22. In the start position, a constant tension magazine spring 24 urges the stack of test strips against the feed barrel 4 so that a single test strip 16 lies flat in the recess 12. Only a single spring is necessary, although an optional second spring is also illustrated.

When the user initiates turning of the feed barrel 4 in the start position (Figures 2a, 3a, 4a), the first thing that happens is that the support collar 22 is moved away from the feed barrel 4 so as to disengage the free end of the retractable sealing member 20 from the groove 26, thereby permitting rotation of the feed barrel 4 (Figures 2b, 3b, 4b).

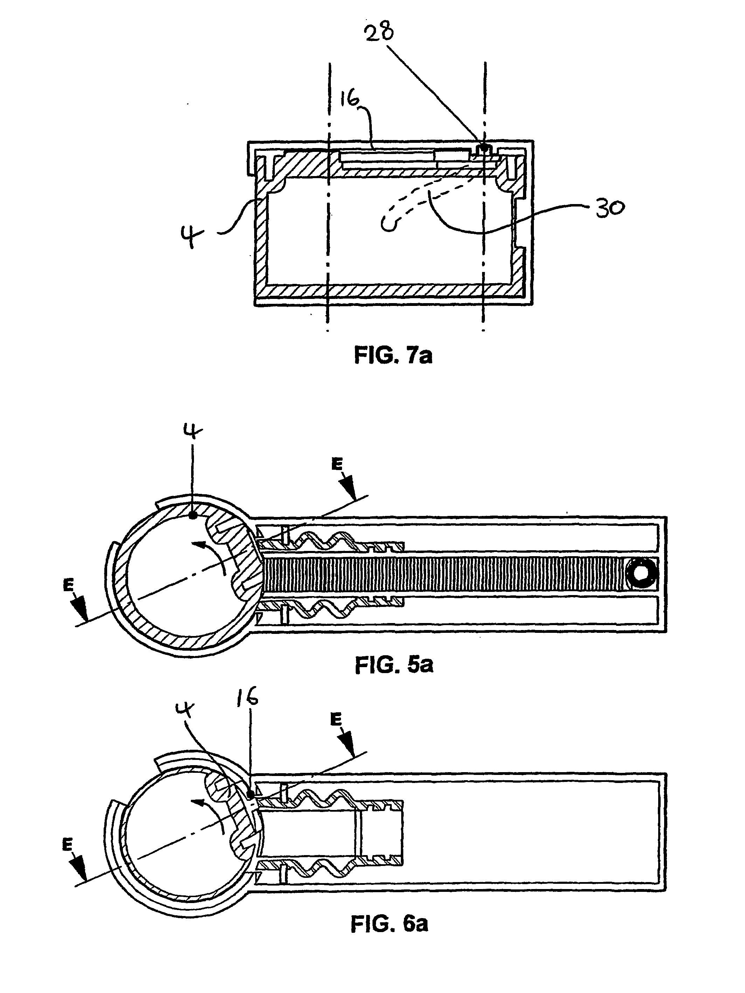

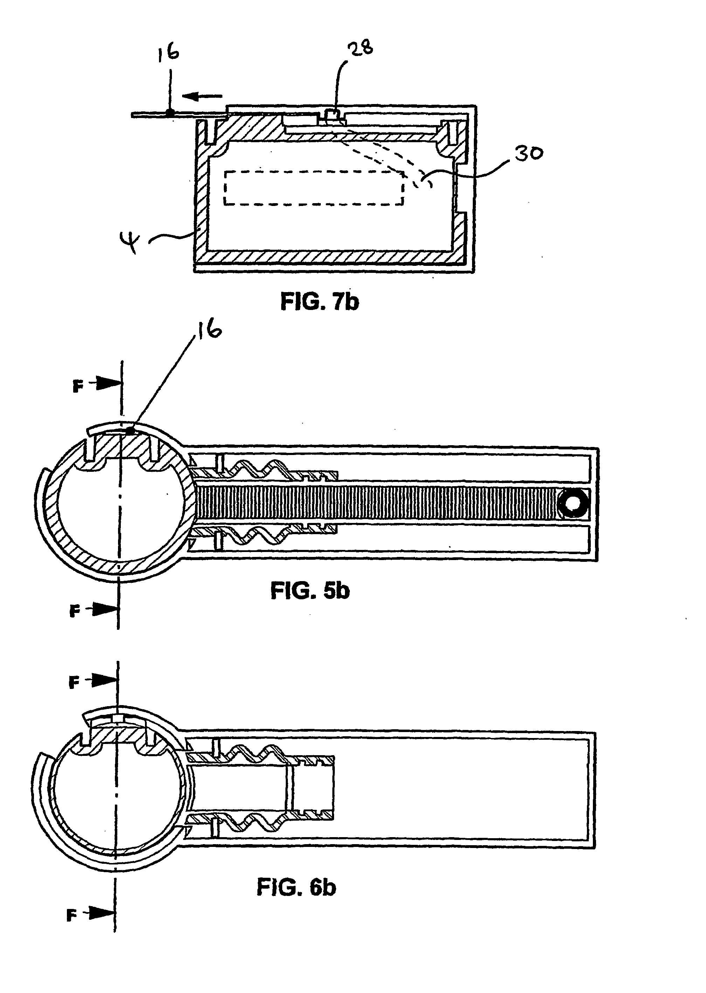

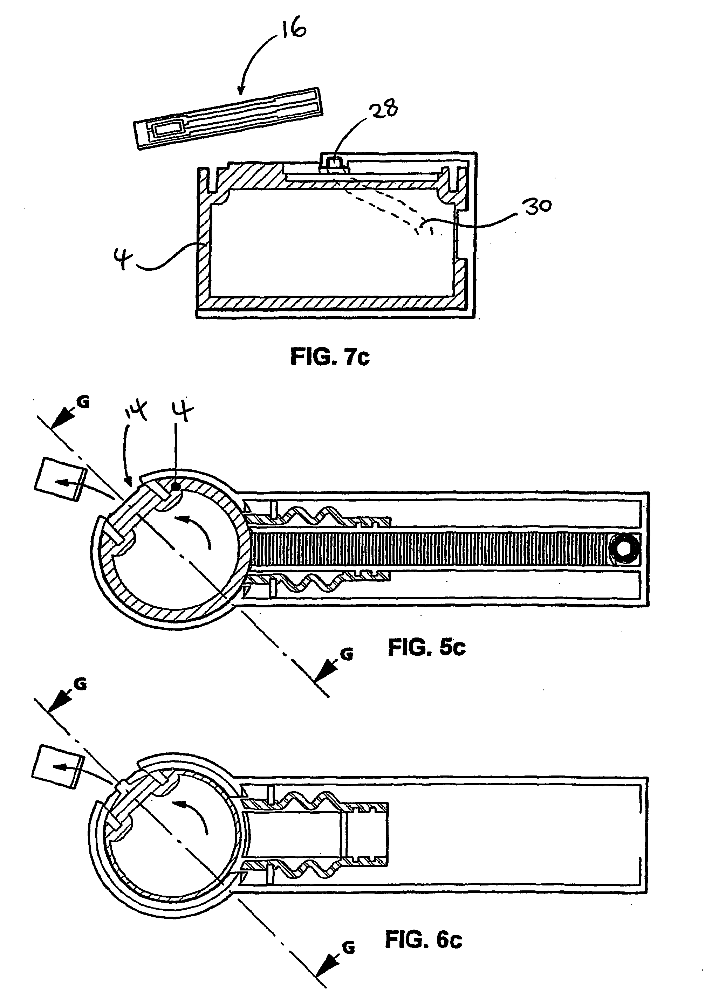

With the retractable sealing member 20 in a retracted configuration, the feed barrel 4, carrying a single test strip 16, is turned by the user (Figures 5a, 6a) until the test strip 16 is at the top of the feed barrel 4, as shown in Figures 5b and 6b. At the same time, a captured pusher 28, behind the test strip 16, engages a helical track 30 (Figures 7a, 7b) in the housing 2. This track 30 moves the pusher 28 forward which in turn moves the test strip 16 out of the housing 2 and into the engagement location. As the test strip 16 reaches the engagement location, it flexes a contact (not shown) into a "wake up" position which activates the meter into a ready for use state. The feed barrel 4 is turned against a return spring (not shown). In the engagement location, the barrel 4 is held by a "ball point pen" type mechanism (not shown). After a reading has been taken, further turning of the feed barrel 4 (Figures 5c, 6c, 7c) brings the recess 12 and used test strip 16 to the opening 14 where the test strip 16 may drop out or urged out by ejection means. On release of the feed barrel 4 from the exit location, the return spring returns the feed barrel 4 to the start position, where the next test member 16 from the stack engages in the recess 12. The process can then be repeated until all test members have been used up. The device may then be discarded.

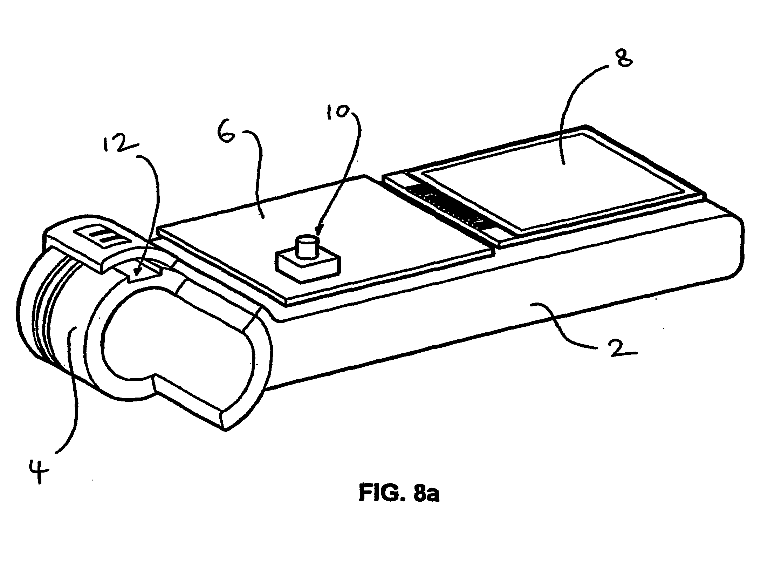

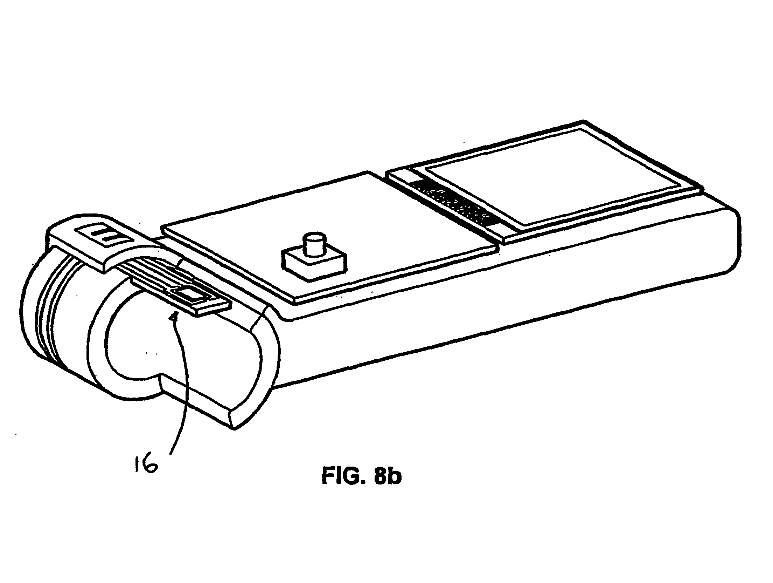

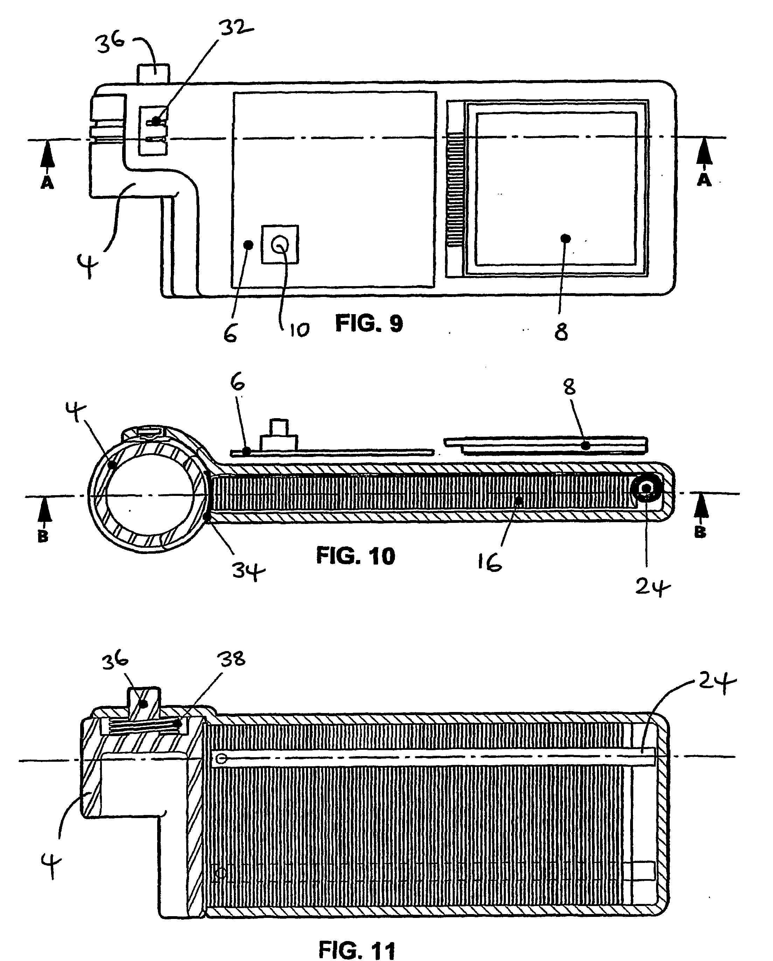

In the embodiments shown in Figures 8 to 19, parts with similar structure and function to the first embodiment are given the same reference number.

In the second embodiment shown in Figures 8 to 13, the feed barrel 4 is partially cutaway so that a test strip 16 is only partially supported in the recess 14. The part of the housing 2 in which the feed barrel 4 is mounted is also cutaway, so that a used test strip which is turned so that it exits the housing can drop out or be ejected. A seal 34 is provided on part of the full length outer surface of the feed barrel 4 so that, in the start position illustrated in Figures 8a and 9 to 11, the seal 34 creates a moisture tight seal between the test strips 16 and the feed barrel 4.

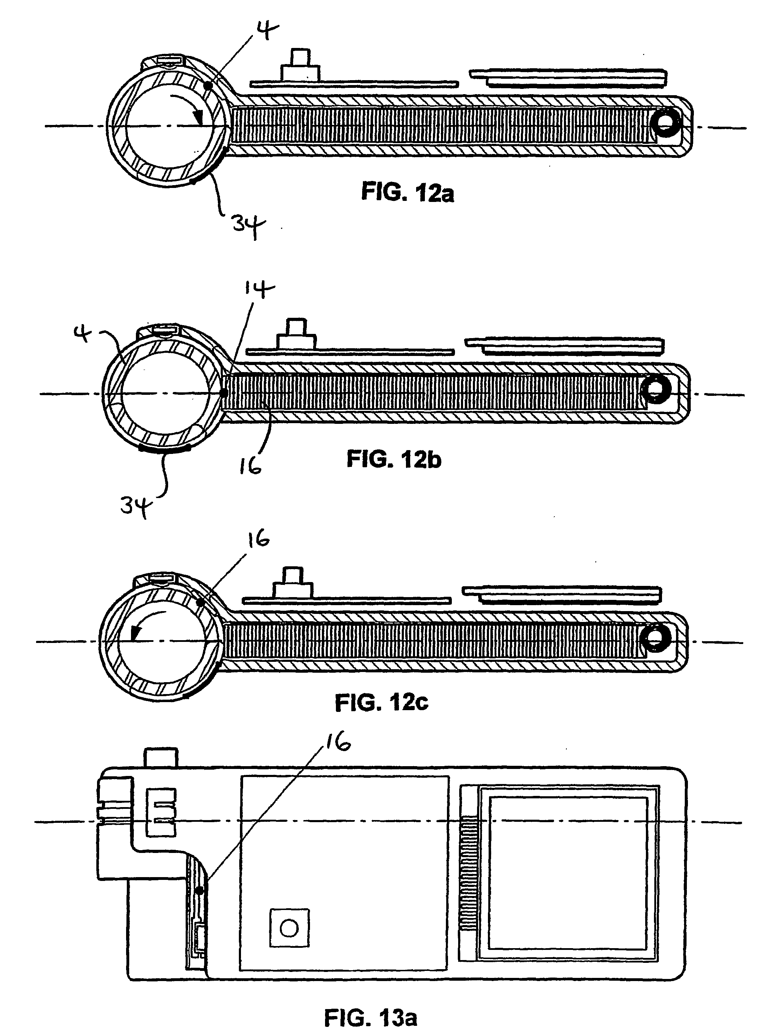

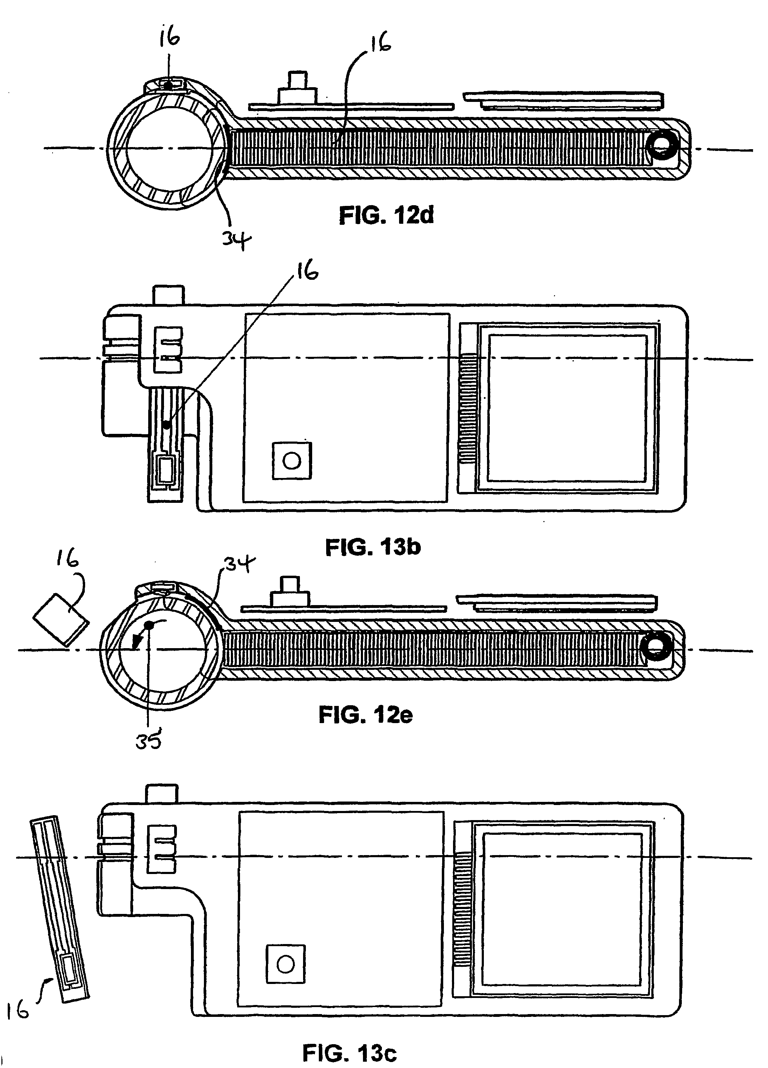

From the start position, a user brings a test strip 16 to the engagement location where it engages with electrical contacts 32 on the housing 2 by the steps illustrated in Figures 12a to 12d, 13a and 13b. The feed barrel 4 is provided with a drive axle 36. The user turns the drive axle 36 in the direction of the arrow as shown in Figure 12a, against the force of a return spring 38, to the feed position shown in Figure 12b. Here the recess 12 is brought into register with the test strips 16, and a single test strip 16 locates in the recess 12. The user then releases the drive axle 36, and the return spring 38 carries the feed barrel 4 back to the start position via the intermediate position shown in Figures 12c and 13a. With the electrode tracks on the test strip 16 engaged with the electrical contacts 32 of the meter, the user now takes a reading of blood glucose concentration. The seal 34 once again seals off the test strip cartridge from atmospheric moisture, so that test strips in the cartridge are protected from moisture while the reading is being taken.

After the reading has been taken, the user turns the drive axle 36 in the direction of the arrow 35 (Figure 12e) and the used test strip 16 is either permitted to drop from the recess 12 or is positively ejected (Figures 12e, 13). The user then releases the drive axle 36 and the feed barrel is returned to the start position by the return spring 38. The cartridge of test strips is kept sealed from moisture at all times except for short periods when a test strip is being loaded and when the used test strip is being ejected.

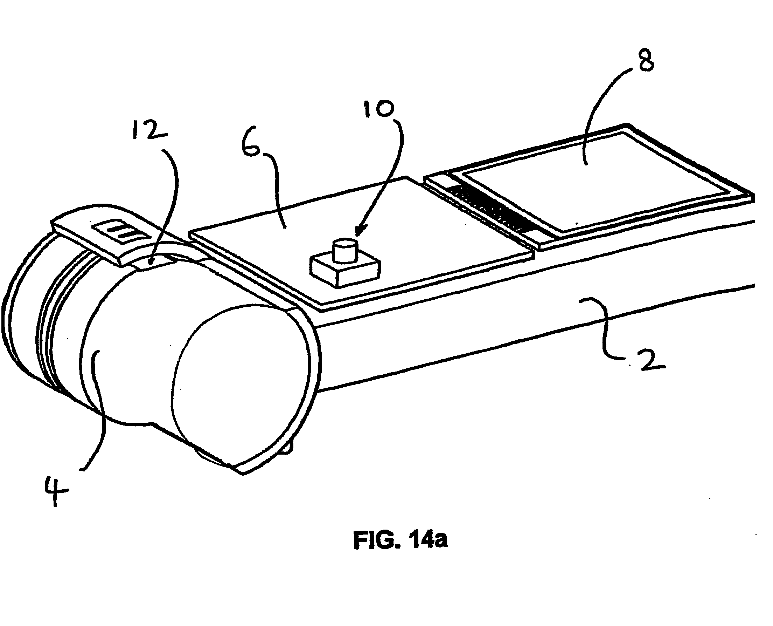

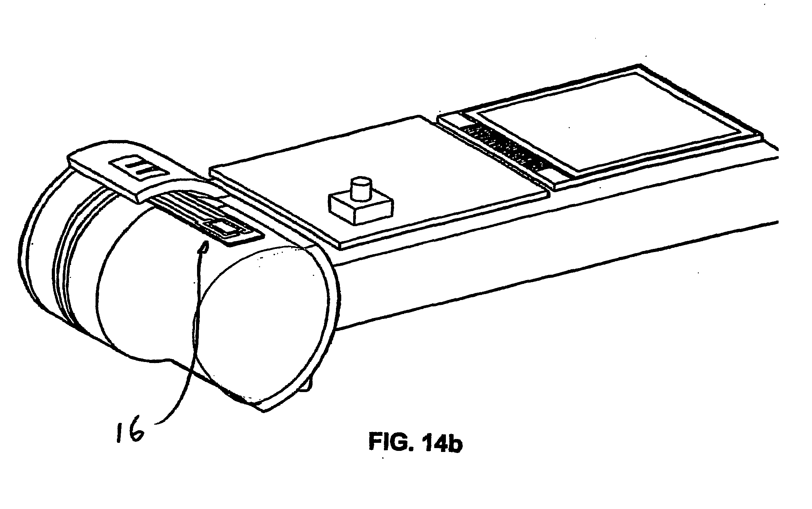

Turning now to Figures 14 to 19, the third embodiment illustrated is similar to the second embodiment, but includes special means for retaining sensors in the magazine and for sealing the magazine even during ejection of a used test strip.

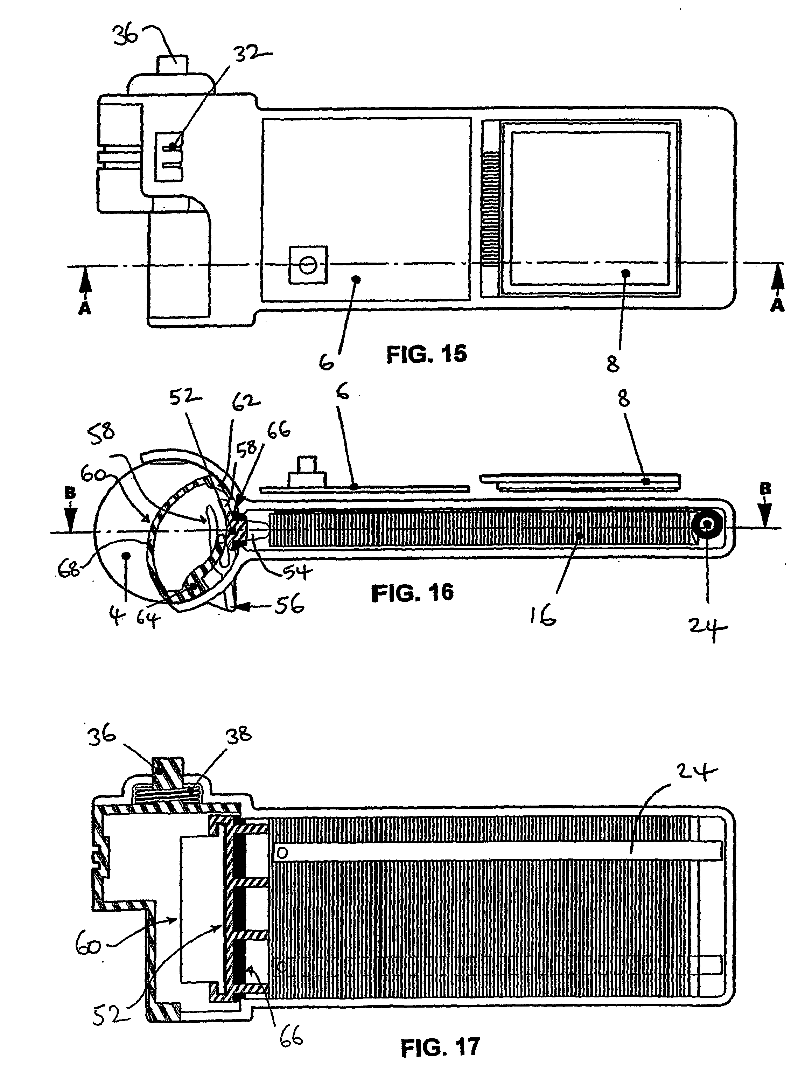

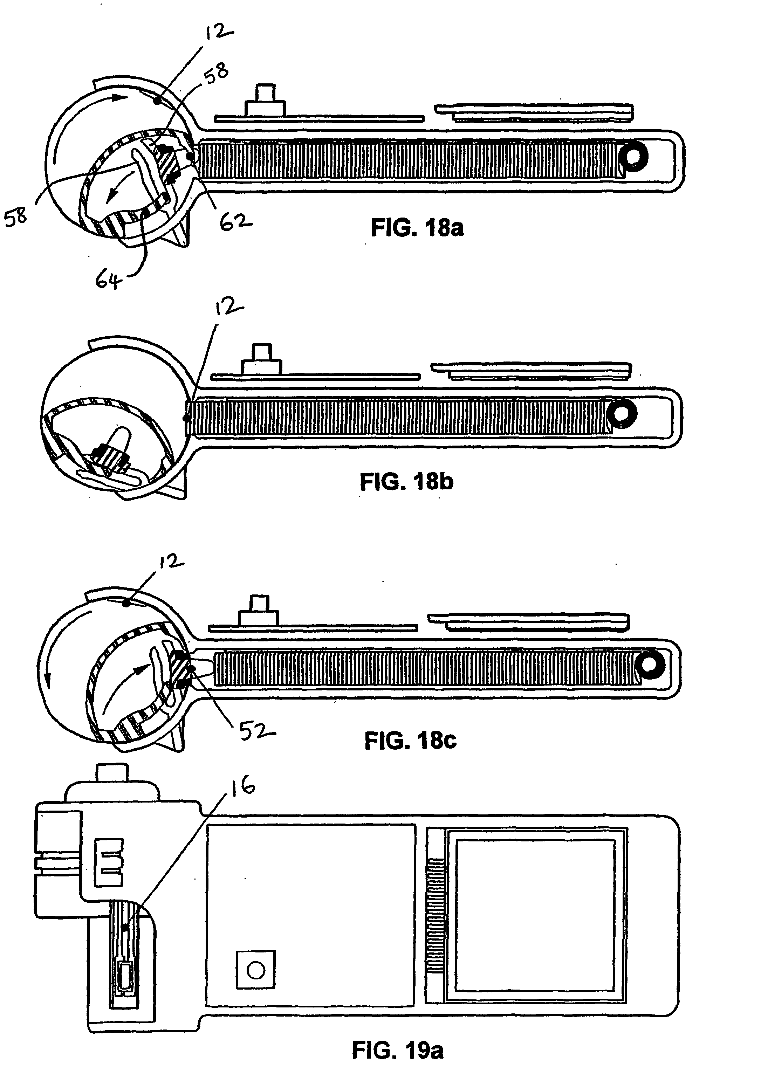

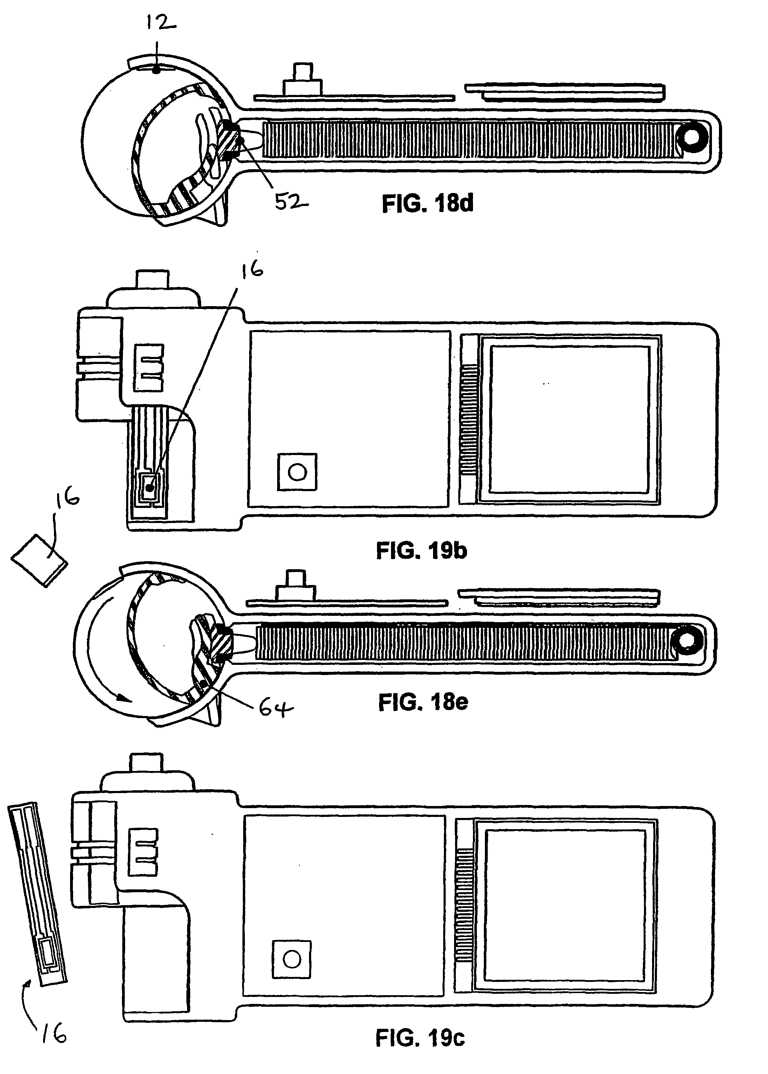

The feed barrel 4 is provided with a separate magazine door 52 which has a door seal 66. When the door 52 is closed, the door seal 66 protects test strips 16 from atmospheric moisture. A plurality of fingers 54 are provided on the door, and these fingers keep the test strips 16 away from the door seal 66. The door 52 is pivotally mounted to a pivot point 56, and is provided with arms 58. Pulling or pushing on an arm 58 causes pivoting of the door 52. Within the feed barrel 4 there is provided an actuating member 60 which includes a pair of spaced apart arms 68 and a pair of spaced apart fingers 64. The end of each finger 64 is journalled between a pair of arms 58 on the magazine door 52. At the end of each arm 68 of the actuating member there is provided a blade 62. To take a blood glucose reading, the following actions are performed. From the start position shown in Figure 16, the user turns the feed barrel 4 in the direction of the arrow shown in Figure 18a. The actuation member 60 also turns, and the fingers 64 pull on an arm 58 of the magazine door 52. The door 52 pivots and begins to withdraw from the magazine. The stack of test strips 16 follows, urged by the spring 24 and restrained by the teeth 54. At the same time, the blades 62 are rotated so that they lie between the teeth 54 and take over the job of restraining the test strips 16 (Figure 18a). As the feed barrel 4 is further rotated (Figure 18b) the blades 62 move out of line with the magazine opening and the recess 12 on the feed barrel moves into alignment with the test strips 16. A single test strip 16 lies flat in the recess 12. The user then permits the return spring 38 to turn the feed barrel 4 in the direction of the arrow shown in Figure 18c, so as to bring the test strip 16 in the recess 12 to the engagement location (Figure 18d). As the barrel 4 turns, initially the blades 62 and subsequently the teeth 54 push the stack of test strips 16 further back into the magazine. The magazine door 52 is closed again and the door seal 66 again seals the inside of the magazine from moisture. After a test reading has been taken, the user turns the feed barrel in the direction shown by the arrow in Figure 18e, and the used test strip is discarded. During testing and during discarding of the test strip, the door 52 remains closed by virtue of lost motion between the fingers 64 on the actuating member 60 and the arms 58 of the door 52. On release of the drive axle 36, the return spring 38 restores the device to the start position and the process can begin again.

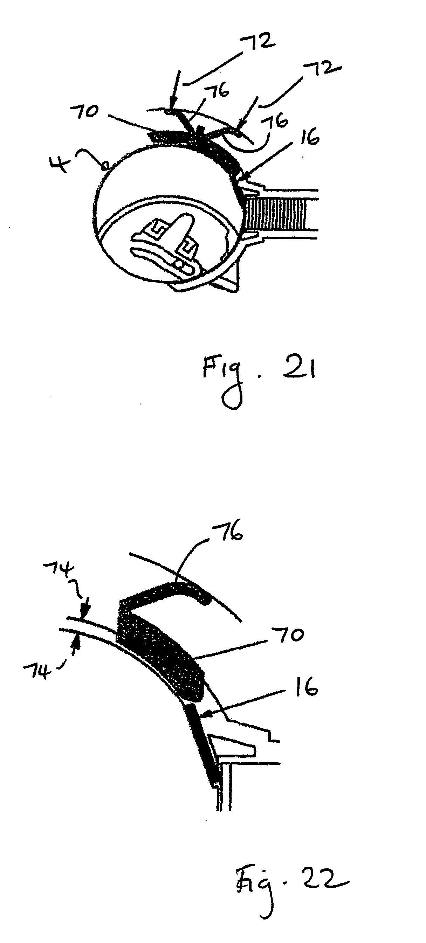

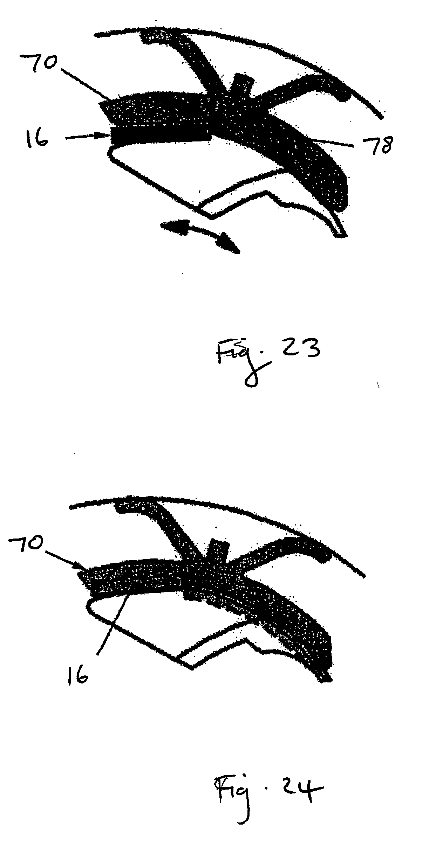

Referring now to Figures 21 to 24, an embodiment is illustrated in which a sliding stop member 70 is provided to help compensate for bowed strips 16. The sliding stop member 70 is provided between the outer surface of the feed barrel 4 and the outer wall of the meter. As the barrel 4 rotates anti-clockwise, bringing a test strip 16 out of the magazine, the leading edge of the stop member 70 is lifted by the edge of the strip 16. As the barrel 4 rotates the strip 16 pushes arms 76 of the sliding stop 70 up against the outer wall creating a low spring force applied to the edges of the strip. The spring force (in the direction of arrows 72) presses the strip 16 down against its recess in the barrel 4 to prevent bowed strips from sticking or jamming the mechanism during use. Figure 22 shows the lead-in 74 designed into the leading edge of the sliding stop 70. The height of the lead-in from the base of the strip recess in the barrel is 1.2 mm. The strip thickness is 0.63 mm (averaged over 50 strips); this means that the sliding stop mechanism can compensate for a maximum strip bow of 1.2-0.63 = 0.57 mm. We have found that typically test strips will have a maximum bow of 0.13 mm.

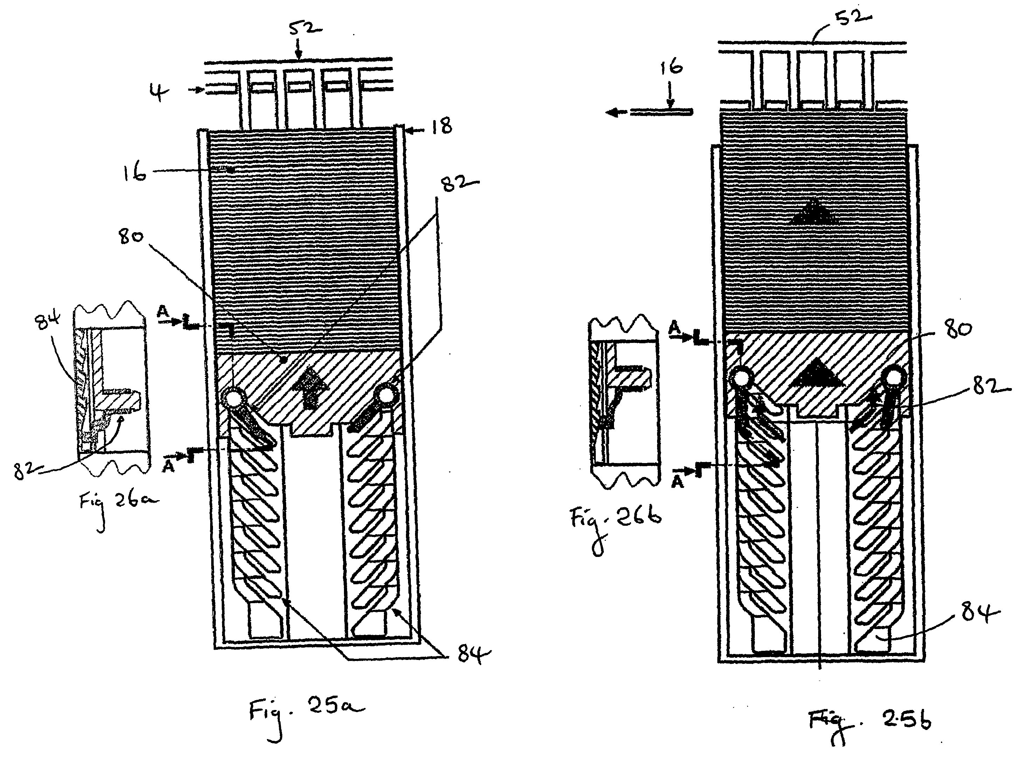

Figure 23 shows the location of the test strip 16 while in use. The strip 16 is located horizontally under the contacts and held in place by the stop member 70. Built into the stop member 70 is a non-return feature 78 that springs down as the strip 16 reaches the engagement location. If the user accidentally turns the barrel 4 in the wrong direction (ie, rotating the barrel to the test member pickup position instead of the eject position) the barrel 4 is free to rotate, which prevents damage to the mechanism, but the test strip 16 will be prevented from moving by the non-return feature 78. This helps prevent contamination of unused test strips with blood from the used strip. Referring now to Figure 24, when the test strip 16 has been ejected, the sliding stop member 70 is in its rest position in a recess in the barrel 4. In this position it covers the front of the electrical contacts preventing ejected strips from being re-inserted into the meter.

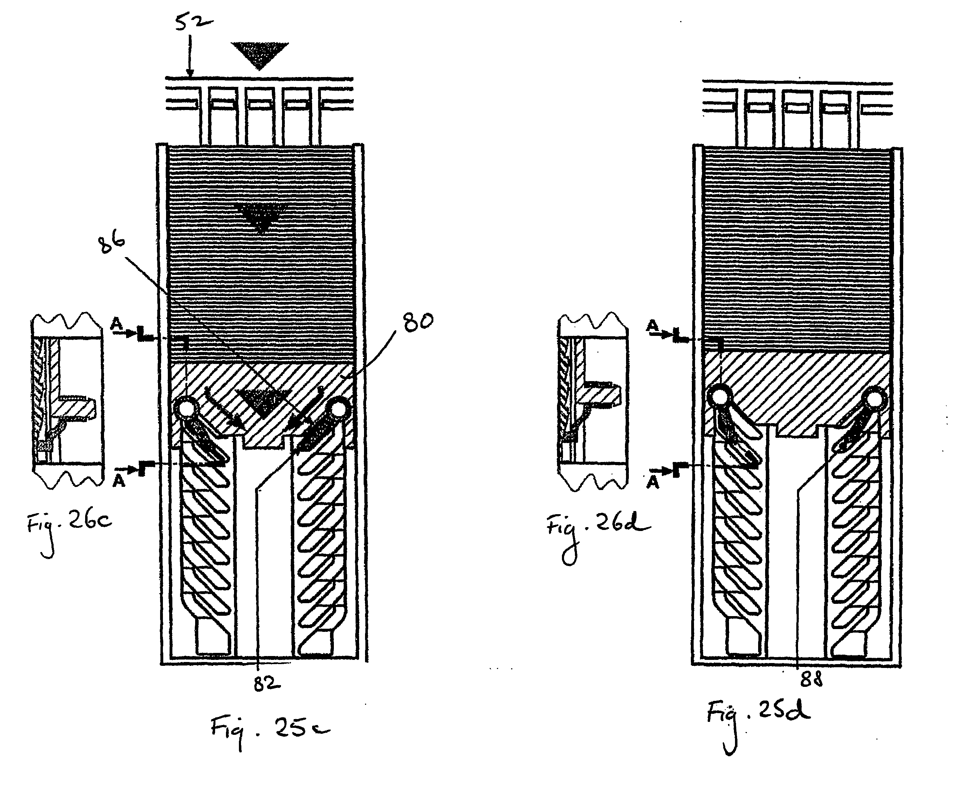

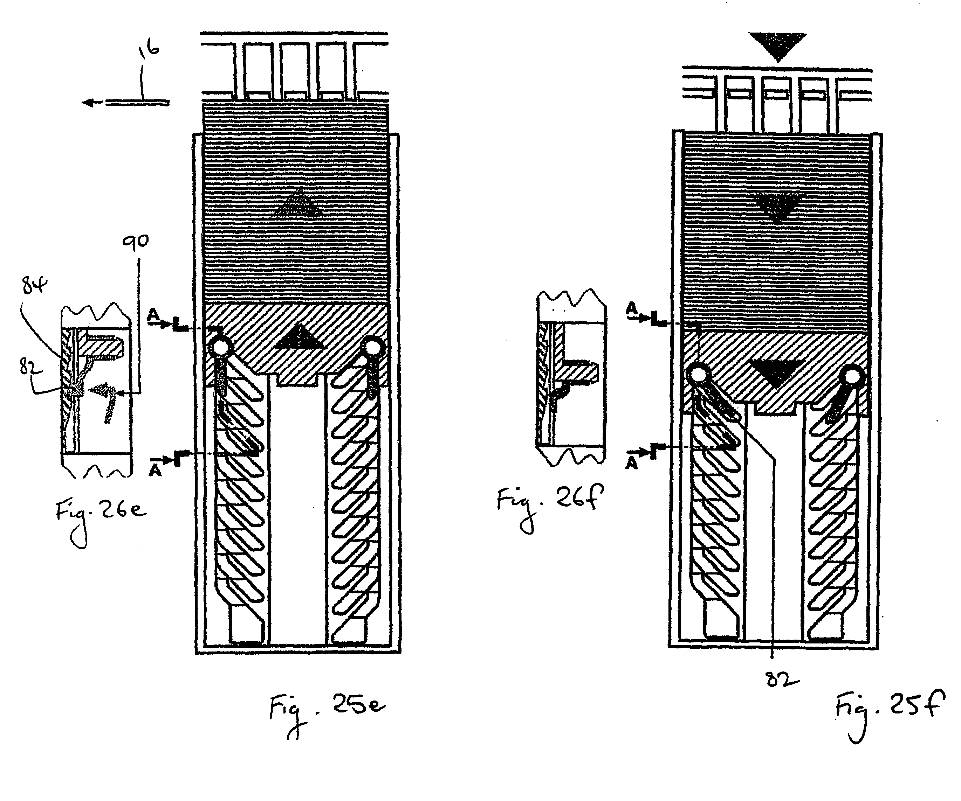

Test strips 16 in the magazine 18 are urged towards the transport member 4 by a follower 80 which is acted on by spring means 24. It is desirable to reduce the backward movement of the follower in the magazine sub-assembly to a distance which is too small to permit the test strips 16 to flip over. A magazine ratchet mechanism to achieve this is illustrated in Figures 25 and 26. Figures 25a and 26a show sections through a magazine 18 which has a stack of test strips 16 of which about half have been used and ejected. The stack is urged by a follower 80 towards the door 52 by a spring (not shown). The follower 80 is provided with a pair of pivotally mounted pawls 82, each of which is engaged in a herringbone ratchet track 84.

Turning to Figures 25b and 26b, retraction of the hinged door 52 permits the stack of test strips 16 and the follower 80 to advance. The twin pawls 82 accommodate the advance of the follower 80 by rotating to the outer edge of the herringbone track, and a test member 16 is extracted from the top of the stack and presented for use. After the test strip 16 is extracted (Figures 25c and 26c), the door 52 is closed and pushes the test member stack and follower 80 down the magazine 18. The right hand pawl 82 prevents the follower 80 from travelling down the magazine 18 because the end of the pawl 82 can go no further than the end of the "bone" of the herringbone track in which the pawl is engaged. Moreover, the side 86 of the follower 80 adjacent the right hand pawl 82 limits rotation of that pawl and so the downward travel of the follower 80.

Figures 25d and 26d show the situation after about seven test strips 16 (dependent on strip thickness) have been extracted since Figures 25c and 26c. The gap 88 represents the longest downward travel the follower 80 is allowed to make before the left hand pawl 82 is snapped into a new herringbone track. Turning now to Figures 25e and 26e, a further test strip 16 is extracted, permitting the left hand pawl 82 to move over the separating wall between two herringbone ratchet tracks so that it snaps down into the next track. The left hand pawl 82 is now the limiter of downward movement for the follower 80 (Figures 25f and 26f) until a further seven test strips 16 have been extracted when the right hand pawl will again take over.

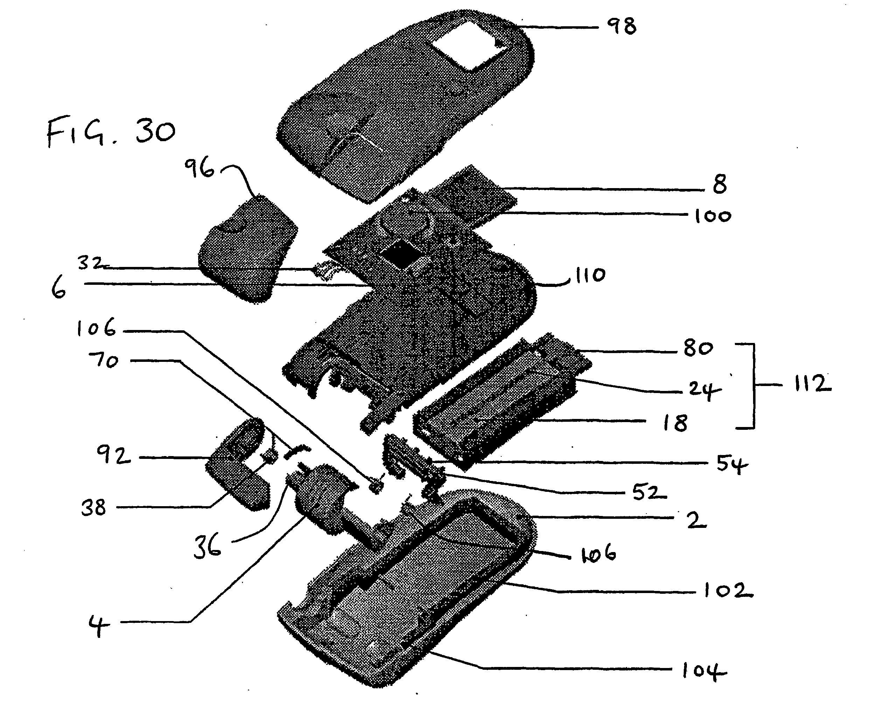

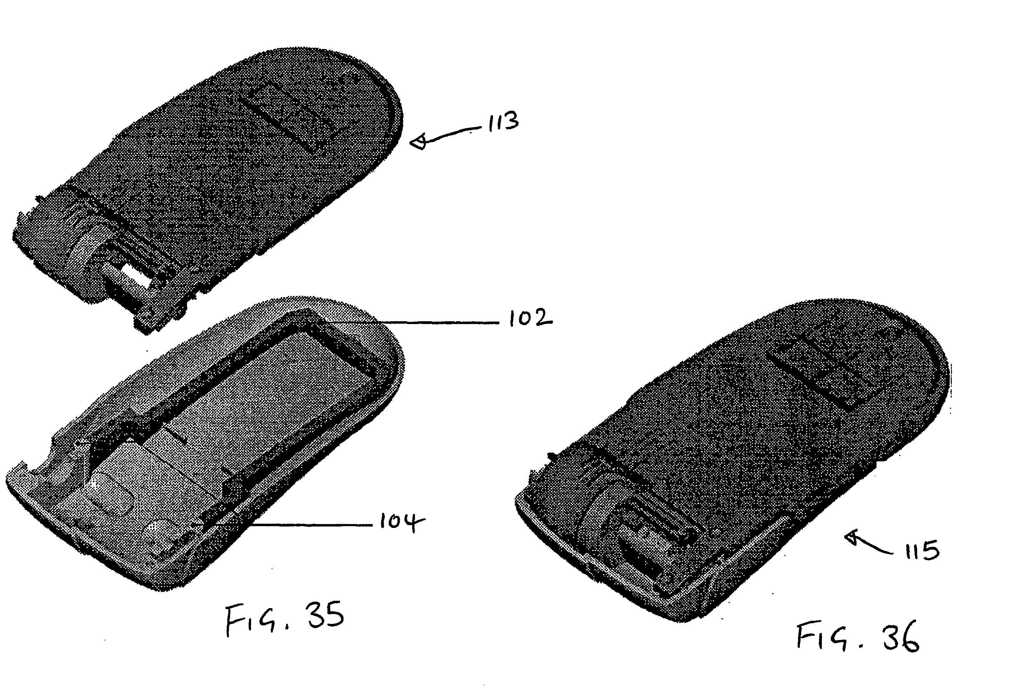

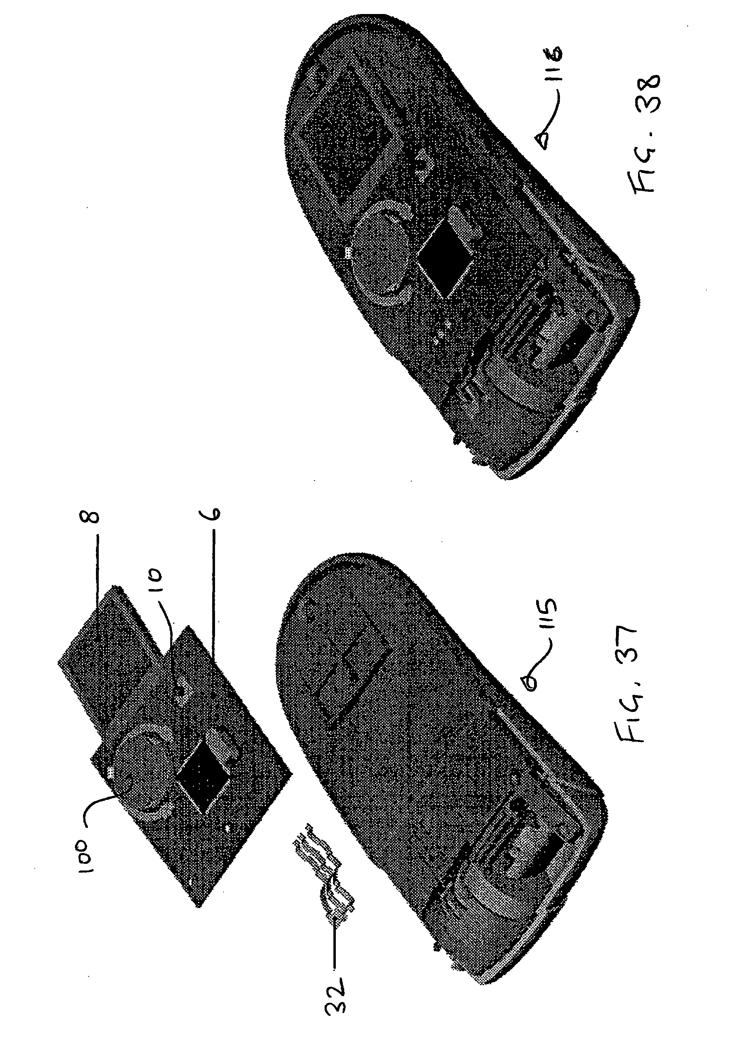

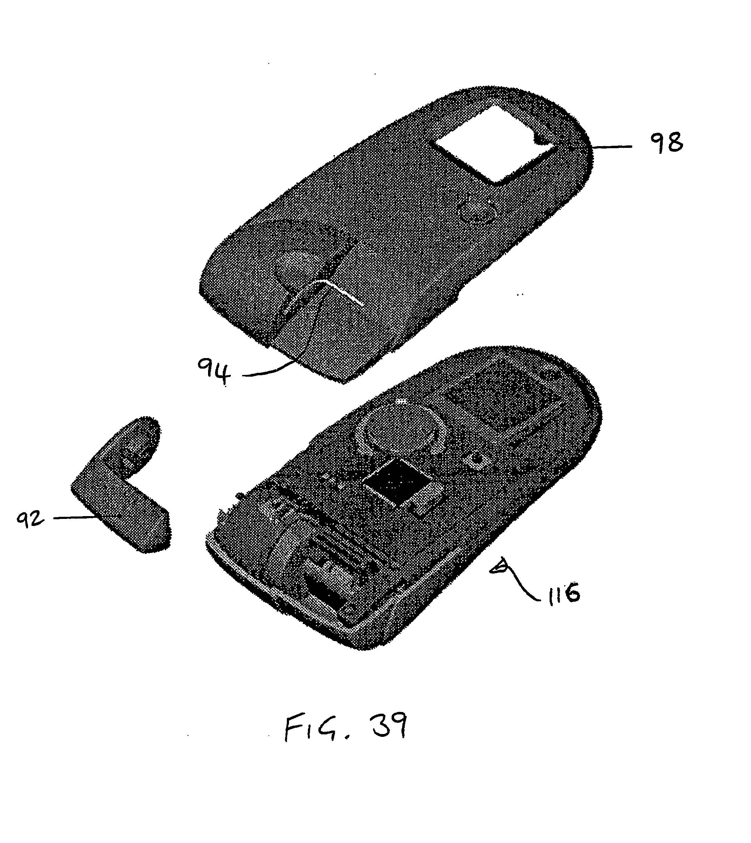

Referring now to Figures 27 to 39, a further alternative embodiment of the invention, and its assembly, is described. The test device has a handle 92 for turning the transport member 4 (best shown in Figure 30)and a slot 94 in its external housing to permit presentation of a test strip. A clip-on dust sleeve 96 covers the slot 94 when the device is not in use.

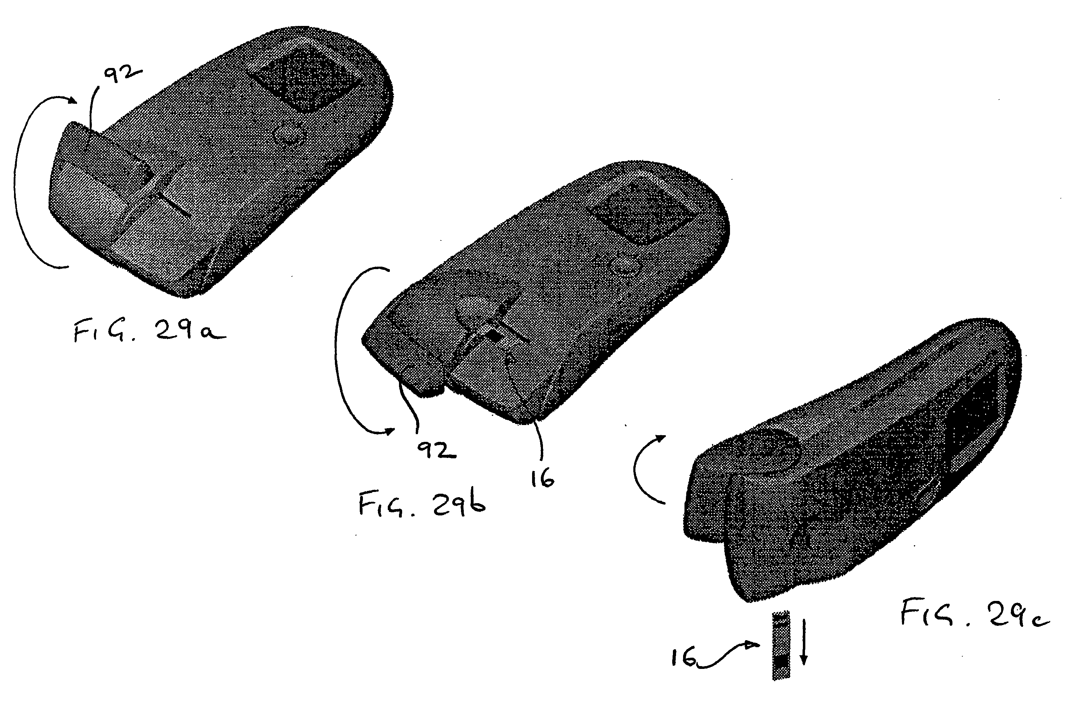

Operation of the device by a user is illustrated in Figure 29. The user pivots the handle 92 upwards (Figure 29a) causing a test strip 16 to be picked up by the transport member. When the handle 92 is returned to its rest position (Figure 29b) a test strip 16 passes through the slot 94 and is presented for use. After a reading has been taken, the handle 92 is turned again (Figure 29c) in the opposite direction to its initial turning, to release the used test strip 16. After the handle is returned to the rest position the device is ready for use again.

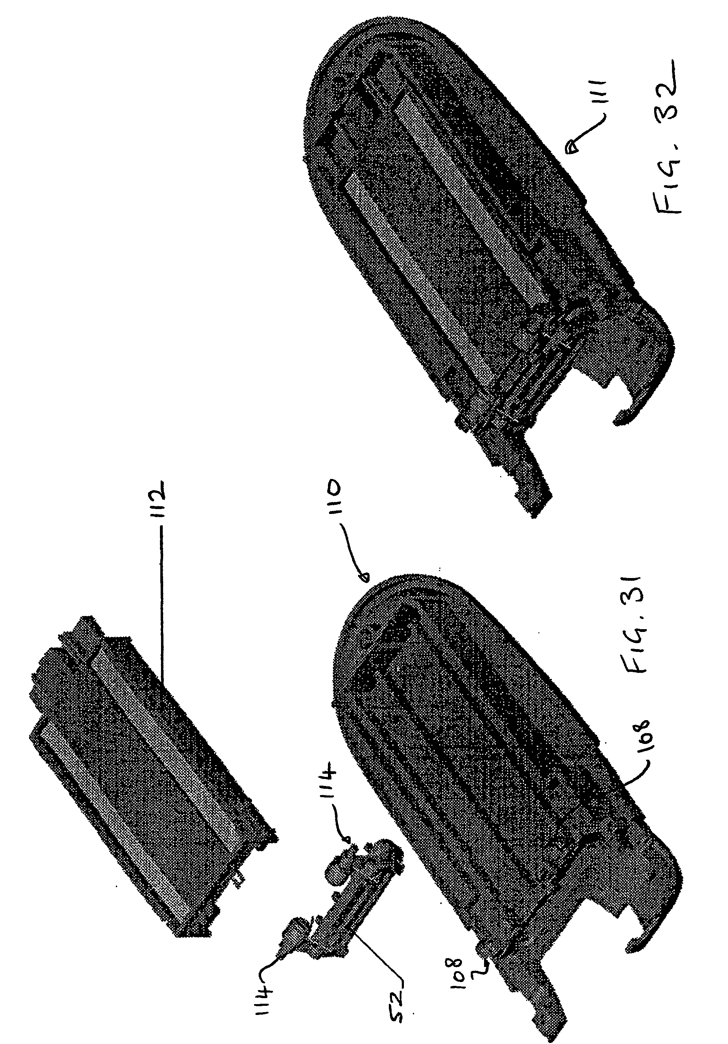

An exploded diagram of the component parts of the device is shown in Figure 30, and their assembly is shown in the subsequent figures. The magazine (first) subassembly 112 comprises the magazine 18 with a stack of test strips therein, the follower 80 and a constant tension spring 24 operatively attached thereto. The magazine subassembly 112 is mounted in the magazine housing member 110. The door 52 and associated seal is pivotally mounted to the magazine housing member 110 by means of pivot pins 114 on the door 52 that are a snap fit for resilient brackets 108 on the magazine housing member 110, as best shown in Figures 31 and 32. The door is spring biased by return springs 106.

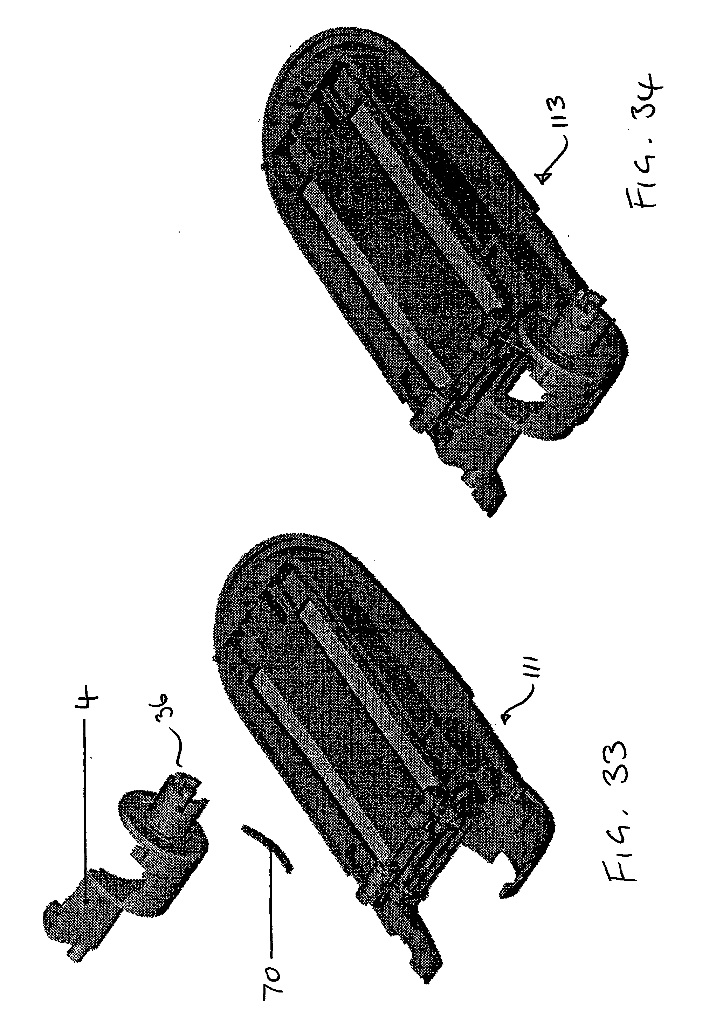

To the resultant door and magazine (second) subassembly 111 is mounted the transport member 4 as best shown in Figure 33. The sliding stop member 70 is mounted between the transport member 4 and the corresponding portion of the magazine housing member 110. This third subassembly 113 is shown in Figure 34.

The third subassembly 113 is then turned over (from the view shown in Figure 34) and secured to the bottom of the housing 2. A gasket 102 is provided in the bottom of the housing 2 to make a seal around the magazine subassembly 112. Stakes 104 are provided on the housing 2 and the magazine housing member 110 which are ultrasonically welded together during assembly of the test device to produce a fourth subassembly 115. Sensor contacts 32 are located appropriately on the fourth subassembly, and the PCB 6 is then mounted on the fourth subassembly 115 so as to retain the sensor contacts 32 in electrical contact therewith. The PCB 6 is provided with appropriate electronics, an LCD 8, battery 100 and memory button 10.

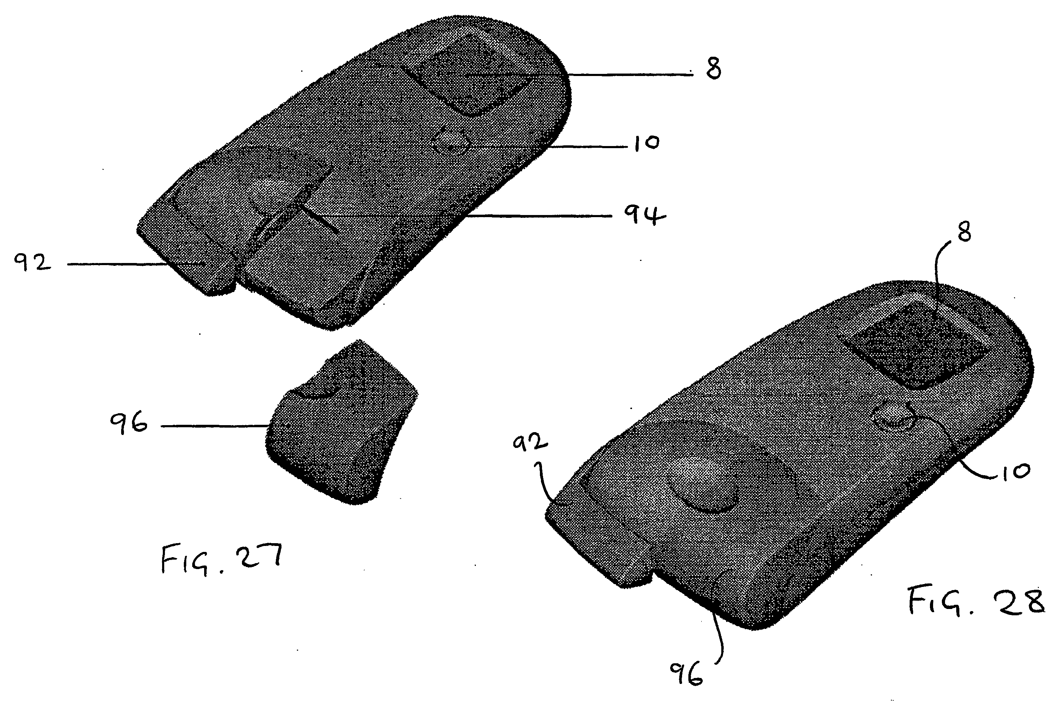

To the resulting final (fifth) subassembly 116 is added a clip-on cover 98, clip-on dust sleeve 96, and the handle 92, which is a push fit for the drive axle 36. A barrel return spring 38 is mounted between the handle 92 and the magazine housing 110 to bias the handle to the rest position shown in Figure 28.

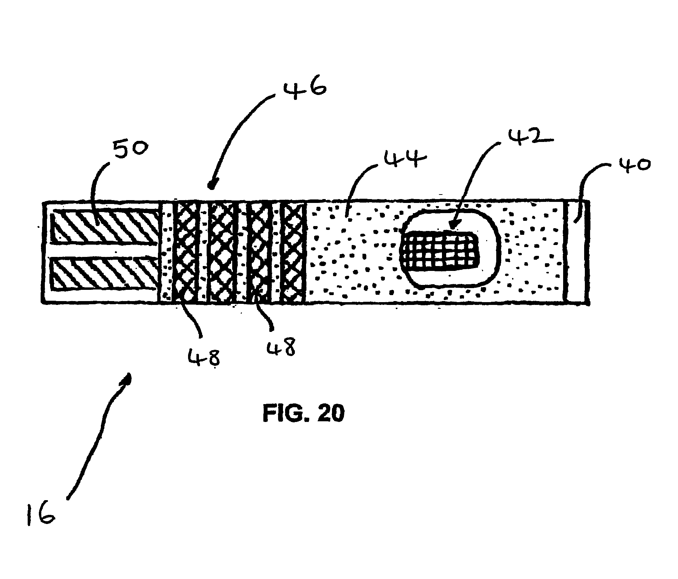

The test strip 16 shown in Figure 20 comprises a planar base member 38, in this example of poly(butylene terephthalate) (PBT) (Valox® FR-1 from GE Plastics). The strip is 30 mm x 5.5 mm, and 0.5 mm thick. A working area 40 is of conventional construction, comprising a plurality of electrodes, a reagent layer in intimate contact with the electrodes, and a mesh layer for spreading out a drop of fluid to be received on the working area. Electrode tracks 48, for example of carbon, in the non-working area 44 of the test strip are connected to the electrodes in the working area 40 in known manner. Also in known manner, a dielectric layer 42 is printed around the working area 40 so as to overlie a portion of the electrode tracks 48, leaving just the ends of the tracks exposed for connection to corresponding electrodes 32 on the meter. The layers are applied to the base member as inks, by screen printing. Each ink layer is about 10 to 20 *µ*m thick, and the mesh is about 59 to 67 *µ*m thick. The working area 40 has a total thickness which is about 100 *µ*m thicker than the non-working area 44 up to the dielectric layer 42.

To increase the thickness of parts of the non-working area, a high relief ink 46 has been printed in four strips. The high relief ink has a dried thickness such that the total thickness of the non-working area to which the high relief ink 46 has been applied is slightly greater than the total thickness of the test strip in the working area 40. Thus, when a stack of such test strips 16 is formed, and a compressive load is applied to the stack by the spring 24, the working area 40 will not bear all the compressive load. Scuffing of the test area will be reduced compared to a conventional test strip in which the working area stands proud of the non-working area.

Although this embodiment has been illustrated with reference to the use of a high relief ink printed in strips, it will be understood that it is not limited to this embodiment. The ink could be printed as a continuous block, and it could entirely surround the working area if desired. Instead of, or in addition to, the high relief ink, other means could also be provided to increase the thickness of the non-working area, for example: an applied pad or tape; embossing of the base layer or an intermediate layer; or an extension of the mesh layer from the working area into the non-working area.

It is appreciated that certain features of the invention, which are for clarity described in the context of separate embodiments, may also be provided in combination in a single embodiment. Conversely, various features of the invention which are, for the sake of brevity, described in the context of a single embodiment, may also be provided separately or in any suitable subcombination.

Although the invention has been described with reference to a test device for measuring blood glucose concentration, it is to be understood that the invention is not limited to this application. The invention may be used in the determination of any analyte in a fluid by the use of suitable reagents in the test strip. Such reagents are well known to those skilled in the art.

## Claims

1. A test device for testing of analyte concentration in a fluid to be applied thereto, the device comprising:
a) a plurality of sensors (16) arranged in a stack, each of said sensors (16) carrying reagent means for producing an electrical signal in response to the concentration of analyte in an applied fluid, each of said sensors (16) having a plurality of electrode tracks for transmitting said electrical signal;
b) a housing (2) having an opening therein and containing the said stack of sensors (16);
c) electrical contacts (32) mounted in relation to the housing (2) for engaging with electrode tracks on a sensor (16) at an engagement location;
c) a meter connected to the said electrical contacts (32), having electronics (6, 8) means for producing a signal output which is dependent on the electrical signal from a sensor (16) when the sensor (16) is engaged with the said contacts; **characterised by**:
d) a transport member (4) rotatably mounted in the opening of the housing (2), having an axis of rotation which spans the opening and having an outer surface which is provided with a recessed region (12) adapted to receive a single sensor (16) from the stack;
e) spring means (24) within the housing (2) which urge the stack of sensors (16) towards the transport member (4) and which urge a single sensor (16) into the said recess (12) when the recess (12) is suitably aligned adjacent to the stack;
f) sealing means (20, 66) for making a moisture tight seal between the transport member (4) and the stack when the transport member (4) is in a specified rotational position; and
g) wherein rotation of the transport member (4) with a sensor (16) in the recessed region (12) will transport the sensor (16) to the engagement location or to a position where the sensor (16) can be moved to the engagement location, whereby electrode tracks (50) of the sensor (16) can engage with the said electrical contacts (32).

2. A test device as claimed in claim 1, wherein the sensors (16) are stacked in a magazine (18) within the housing (2), the magazine (18) having a single opening which faces the transport member (4).

3. A test device as claimed in claim 2, wherein a first end of the sealing means (20) forms a seal around the magazine (18) and a second end of the sealing means (20) locates in a groove (26) in the transport member (4) to form a seal therewith when the recessed region (12) of the transport member (4) is in register with the stack of sensors (16).

4. A test device as claimed in claim 3, wherein the sealing means comprises a retractable sleeve (20) which sealingly engages in the groove (26) of the transport member (4) when in an extended configuration and which does not form a seal with the transport member (4) when in a retracted configuration.

5. A test device as claimed in any one of the preceding claims, wherein a pusher (28) is provided to impart translational motion to a sensor (16) mounted in the said recessed region (12) during and/or after rotation of the transport member (4) so as to bring the sensor (16) to the engagement location.

6. A test device as claimed in claim 5, wherein the pusher (28) is mounted on the transport member (4) and a portion of the pusher (28) is located in a helical track (30) in the housing (2) whereby rotation of the transport member (4) imparts translational motion to the pusher (28).

7. A test device as claimed in claim 1, wherein the said opening is the only opening to the inside of the housing (2), and wherein the sealing means comprises a seal (20) which is secured in relation to an outer surface of the transport member (4) and which seals the opening of the housing (2) when the transport member (4) is in a specified rotational position.

8. A test device as claimed in claim 2, wherein the sealing means comprises a seal (20) which is secured in relation to an outer surface of the transport member (4) and which seals the opening of the magazine (18) when the transport member (4) is in a specified rotational position.

9. A test device as claimed in claim 1, wherein the said opening is the only opening to the inside of the housing (2), and wherein the sealing means comprises a seal (66) provided on a door (52) which is adapted to fit the said opening so that the moisture tight seal is effected by closure of the door (52); wherein the door (52) is operatively connected to the transport member (4) so that the door (52) will be open when the transport member (4) is in a first rotational position and closed when the transport member (4) is in a second rotational position.

10. A test device as claimed in claim 2, wherein the sealing means comprises a seal (66) provided on a door (52) which is adapted to fit the opening of the magazine (18) so that the moisture tight seal is effected by closure of the door (52); wherein the door (52) is operatively connected to the transport member (4) so that the door (52) will be open when the transport member (4) is in a first rotational position and closed when the transport member (4) is in a second rotational position.

11. A test device as claimed in claim 9 or claim 10, wherein the door (52) is provided with one or more teeth (54) which restrain movement of the stack of sensors (16) against the force of the spring means (24).

12. A test device as claimed in claim 11, wherein the transport member (4) is provided with at least one blade (62) which takes over the function of restraining the stack of sensors (16) when the door (52) is opened.

13. A test device as claimed in any one of claims 9 to 12, wherein the door (52) is pivotally mounted in relation to the housing (2).

14. A test device as claimed in any one of the preceding claims, wherein the transport member (4) is operationally connected to a return spring (38) which urges the transport member (4) to adopt a specified rotational position at which the sealing means (20, 66) can provide a moisture proof seal between the stack of sensors (16) and the transport member (4).

15. A test device as claimed in any one of the preceding claims, wherein a portion of the sensor (16) to which a fluid sample is to be applied is not supported by the transport member (4) when in the engagement location.

16. A test device as claimed in any one of the preceding claims, wherein the transport member (4) has an external profile which is substantially circular in cross section.

17. A test device as claimed in any one of the preceding claims, wherein each sensor (16) in the stack comprises a base member having a working area to which the fluid is to be applied, containing the reagent means, and a non-working area (44) adjacent to the working area, wherein the total thickness of the sensor (16) in at least a portion of the non-working area (44) is at least as great as the total thickness of the sensor (16) in the working area.

18. A test device as claimed in claim 17, wherein the total thickness of the sensor (16) in at least a part of the non-working area (44) is greater than the total thickness of the sensor (16) in the working area.

19. A test device as claimed in any one of the preceding claims, further including load means (70) for applying a compressive load to a sensor (16) during at least a part of the time when the said sensor (16) is located in the recessed region (12) of the transport member (4).

20. A test device as claimed in any one of the preceding claims, further including non-return means (78) which prevent or inhibit transport of a sensor (16) from the engagement location to the magazine (18) and which prevent or inhibit reintroduction of an ejected used sensor (16) to the engagement location.

21. A test device as claimed in claim 20, wherein the said non-return means (78) and the said load means (70) comprise a single resilient and flexible component.

22. A test device as claimed in any one of the preceding claims, further including ratchet means (82, 84) associated with the stack of sensors (16) which prevent or inhibit movement of the stack in a direction opposite to that in which the spring means (24) urges the stack.

23. A test device as claimed in any one of the preceding claims, suitable for use in testing glucose concentration in blood.

## Patentansprüche

1. Testgerät zum Testen einer Analytkonzentration in einer darauf aufzubringenden Flüssigkeit, wobei das Gerät Folgendes umfasst:
a) eine Vielzahl von stapelförmig angeordneten Sensoren (16), wobei alle Sensoren (16) ein Reagensmittel zur Erzeugung eines elektrischen Signals als Reaktion auf die Analytkonzentration in einer aufgebrachten Flüssigkeit sowie eine Vielzahl von Elektrodenbahnen zur Übertragung des elektrischen Signals aufweisen;
b) ein Gehäuse (2) mit einer darin befindlichen Öffnung, das den Stapel Sensoren (16) enthält;
c) in Relation zu dem Gehäuse (2) angebrachte elektrische Kontakte (32) zum Eingriff in Elektrodenbahnen auf einem Sensor (16) an einer Eingriffsposition;
c) ein mit den elektrischen Kontakten (32) verbundenes Messgerät mit elektronischen Mitteln (6, 8) zur Erzeugung einer Signalausgabe, die von dem elektrischen Signal von einem Sensor (16) abhängt, wenn der Sensor (16) in die Kontakte eingreift; **gekennzeichnet durch**:
d) ein in der Öffnung des Gehäuses (2) drehbar angebrachtes Transportelement (4) mit einer die Öffnung überspannenden Drehachse und einer Außenfläche mit einem vertieften Bereich (12), der so ausgebildet ist, dass er einen einzelnen Sensor (16) von dem Stapel aufnimmt;
e) Federmittel (24) in dem Gehäuse (2), die den Stapel Sensoren (16) in Richtung des Transportelementes (4) schieben und einen einzelnen Sensor (16) in die Vertiefung (12) schieben, wenn die Vertiefung (12) in geeigneter Weise an dem Stapel ausgerichtet ist;
f) Versiegelungsmittel (20, 26) zur Erzeugung einer feuchtigkeitsdichten Abdichtung zwischen dem Transportelement (4) und dem Stapel, wenn sich das Transportelement (4) in einer speziellen Drehposition befindet; und
g) wobei die Drehung des Transportelementes (4) mit einem Sensor (16) in dem vertieften Bereich (12) den Sensor (16) in die Eingriffsposition oder eine Position, in der der Sensor (16) in die Eingriffsposition bewegt werden kann, transportiert, wobei die Elektrodenbahnen (50) des Sensors (16) in die elektrischen Kontakte (32) eingreifen können.

2. Testgerät nach Anspruch 1, bei dem die Sensoren (16) in einem Magazin (18) in dem Gehäuse (2) gestapelt sind, wobei das Magazin (18) eine einzige Öffnung hat, die dem Transportelement (4) gegenüber liegt.

3. Testgerät nach Anspruch 2, bei dem ein erstes Ende des Versiegelungsmittels (20) eine Abdichtung um das Magazin (18) herum bildet und sich ein zweites Ende des Versiegelungsmittels (20) in einer Rille (26) in dem Transportelement (4) befindet und eine Abdichtung damit bildet, wenn der vertiefte Bereich (12) des Transportelementes (4) an dem Stapel Sensoren (16) ausgerichtet ist.

4. Testgerät nach Anspruch 3, bei dem das Versiegelungsmittel einen einziehbaren Kontaktschutz (20) umfasst, der in ausgezogenem Zustand versiegelnd in die Rille (26) des Transportelementes (4) eingreift und in eingezogenem Zustand keine Abdichtung mit dem Transportelement (4) bildet.

5. Testgerät nach einem der vorangegangenen Ansprüche, bei dem eine Schubvorrichtung (28) vorgesehen ist, um einem in dem vertieften Bereich (12) angebrachten Sensor (16) während und/oder nach der Drehung des Transportelementes (4) eine Translationsbewegung zu verleihen und den Sensor (16) in die Eingriffsposition zu transportieren.

6. Testgerät nach Anspruch 5, bei dem die Schubvorrichtung (28) an dem Transportelement (4) angebracht ist und sich ein Teil der Schubvorrichtung (28) in einer spiralförmigen Bahn (30) in dem Gehäuse (2) befindet, wobei die Drehung des Transportelementes (4) der Schubvorrichtung (28) eine Translationsbewegung verleiht.

7. Testgerät nach Anspruch 1, bei dem die Öffnung die einzige Öffnung zum Inneren des Gehäuses (2) ist und das Versiegelungsmittel eine Abdichtung (20) umfasst, die in Relation zu einer Außenfläche des Transportelementes (4) befestigt ist und die Öffnung des Gehäuses (2) versiegelt, wenn sich das Transportelement (4) in einer speziellen Drehposition befindet.

8. Testgerät nach Anspruch 2, bei dem das Versiegelungsmittel eine Abdichtung (20) umfasst, die in Relation zu einer Außenfläche des Transportelementes (4) befestigt ist und die Öffnung des Magazins (18) versiegelt, wenn sich das Transportelement (4) in einer speziellen Drehposition befindet.

9. Testgerät nach Anspruch 1, bei dem die Öffnung die einzige Öffnung zum Inneren des Gehäuses (2) ist und das Versiegelungsmittel eine Abdichtung (66) an einer Tür (52) umfasst, die so ausgebildet ist, dass sie in die Öffnung passt, so dass durch Schließen der Tür (52) eine feuchtigkeitsdichte Abdichtung erzielt wird, wobei die Tür (52) derart funktionsmäßig mit dem Transportelement (4) verbunden ist, dass die Tür (52) offen ist, wenn sich das Transportelement (4) in einer ersten Drehposition befindet, und geschlossen, wenn sich das Transportelement (4) in einer zweiten Drehposition befindet.

10. Testgerät nach Anspruch 2, bei dem das Versiegelungsmittel eine Abdichtung (66) an einer Tür (52) umfasst, die so ausgebildet ist, dass sie in die Öffnung des Magazins (18) passt, so dass durch Schließen der Tür (52) eine feuchtigkeitsdichte Abdichtung erzielt wird, wobei die Tür (52) derart funktionsmäßig mit dem Transportelement (4) verbunden ist, dass die Tür (52) offen ist, wenn sich das Transportelement (4) in einer ersten Drehposition befindet, und geschlossen, wenn sich das Transportelement (4) in einer zweiten Drehposition befindet.

11. Testgerät nach Anspruch 9 oder 10, bei dem die Tür (52) mit einem oder mehreren Zähnen (54) versehen ist, die eine Bewegung des Stapels Sensoren (16) gegen die Kraft des Federmittels (24) verhindern.

12. Testgerät nach Anspruch 11, bei dem das Transportelement (4) mit mindestens einer Lamelle (62) versehen ist, die die Funktion des Rückhaltens des Stapels Sensoren (16) bei offener Tür (52) übernimmt.

13. Testgerät nach einem der Ansprüche 9 bis 12, bei dem die Tür (52) in Relation zu dem Gehäuse (2) schwenkbar angebracht ist.

14. Testgerät nach einem der vorangegangenen Ansprüche, bei dem das Transportelement (4) funktionsmäßig mit einer Rückstellfeder (38) verbunden ist, die das Transportelement (4) in eine spezielle Drehposition schiebt, in der die Versiegelungsmittel (20, 66) eine feuchtigkeitsdichte Abdichtung zwischen dem Stapel Sensoren (16) und dem Transportelement (4) bereitstellen können.

15. Testgerät nach einem der vorangegangenen Ansprüche, bei dem ein Teil des Sensors (16), auf den eine Flüssigkeitsprobe aufzubringen ist, in der Eingriffsposition nicht auf dem Transportelement (4) lagert.

16. Testgerät nach einem der vorangegangenen Ansprüche, bei dem das Transportelement (4) ein Außenprofil mit einem im Wesentlichen kreisförmigen Querschnitt besitzt.

17. Testgerät nach einem der vorangegangenen Ansprüche, bei dem alle Sensoren (16) in dem Stapel ein Basiselement mit einer Arbeitsfläche, auf die die Flüssigkeit aufzubringen ist und die das Reagensmittel enthält, und einer an die Arbeitsfläche angrenzenden Nichtarbeitsfläche (44) umfassen, wobei die Gesamtdicke des Sensors (16) in mindestens einem Teil der Nichtarbeitsfläche (44) mindestens so groß ist wie die Gesamtdicke des Sensors (16) in der Arbeitsfläche.

18. Testgerät nach Anspruch 17, bei dem die Gesamtdicke des Sensors (16) in mindestens einem Teil der Nichtarbeitsfläche (44) größer ist als die Gesamtdicke des Sensors (16) in der Arbeitsfläche.

19. Testgerät nach einem der vorangegangenen Ansprüche, das weiterhin Lastmittel (70) zur Ausübung einer Drucklast auf einen Sensor (16) während mindestens eines Teils der Zeit, in dem sich der Sensor (16) in dem vertieften Bereich (12) des Transportelementes (4) befindet, einschließt.

20. Testgerät nach einem der vorangegangenen Ansprüche, das weiterhin ein Non-return-Mittel (78) einschließt, das den Transport eines Sensors (16) von der Eingriffsposition zum Magazin (18) sowie die erneute Einführung eines ausgestoßenen gebrauchten Sensors (16) in die Eingriffsposition ver- bzw. behindert.

21. Testgerät nach Anspruch 20, bei dem das Non-return-Mittel (78) und das Lastmittel (70) eine einzige elastische und biegsame Komponente umfassen.

22. Testgerät nach einem der vorangegangenen Ansprüche, das weiterhin mit dem Stapel Sensoren (16) assoziierte Klinkenradmittel (82, 84) einschließt, die die Bewegung des Stapels in eine Richtung, die der Richtung, in die das Federmittel (24) den Stapel schiebt, entgegen gesetzt ist, ver- bzw. behindern.

23. Testgerät nach einem der vorangegangenen Ansprüche, das sich zur Anwendung bei Tests der Glucosekonzentration im Blut eignet.

## Revendications

1. Dispositif d'analyse permettant d'analyser la concentration d'une substance à analyser dans un fluide à appliquer à celui-ci, le dispositif comprenant :
a) une pluralité de capteurs (16) agencés dans une pile, chacun desdits capteurs (16) transportant des moyens réactifs pour produire un signal électrique en réponse à la concentration de substance à analyser dans un fluide appliqué, chacun desdits capteurs (16) comportant une pluralité de pistes d'électrode permettant de transmettre ledit signal électrique ;
b) un boîtier (2) présentant une ouverture dans celui-ci et contenant ladite pile de capteurs (16) ;
c) des contacts électriques (32) installés par rapport au boîtier (2) pour s'accoupler avec des pistes d'électrode sur un capteur (16) à un emplacement d'accouplement ;
d) un lecteur connecté auxdits contacts électriques (32), comportant des moyens électroniques (6, 8) permettant de produire une sortie de signal qui dépend du signal électrique provenant d'un capteur (16) lorsque le capteur (16) est accouplé avec lesdits contacts ; **caractérisé par**
e) un élément de transport (4) installé en rotation dans l'ouverture du boîtier (2), ayant un axe de rotation qui couvre l'ouverture et ayant une surface extérieure qui est munie d'une région renfoncée (12) adaptée pour recevoir un seul capteur (16) de la pile ;
f) des moyens de ressort (24) à l'intérieur du boîtier (2) qui poussent la pile de capteurs (16) en direction de l'élément de transport (4) et qui poussent un seul capteur (16) dans ledit renfoncement (12) lorsque le renfoncement (12) est aligné de façon adéquate à côté de la pile ;
g) des moyens d'étanchéité (20, 66) permettant de réaliser un joint étanche à l'humidité entre l'élément de transport (4) et la pile lorsque l'élément de transport (4) est dans une position de rotation spécifiée ; et
h) dans lequel la rotation de l'élément de transport (4) avec un capteur (16) dans la région renfoncée (12) transportera le capteur (16) jusqu'à l'emplacement d'accouplement ou jusqu'à une position où le capteur (16) peut être déplacé jusqu'à l'emplacement d'accouplement, de manière à ce que les pistes d'électrode (50) du capteur (16) puissent s'accoupler avec lesdits contacts électriques (32).

2. Dispositif d'analyse selon la revendication 1, dans lequel les capteurs (16) sont empilés dans un chargeur (18) à l'intérieur du boîtier (2), le chargeur (18) présentant une seule ouverture qui fait face à l'élément de transport (4).

3. Dispositif d'analyse selon la revendication 2, dans lequel une première extrémité des moyens d'étanchéité (20) forme un joint autour du chargeur (18) et une deuxième extrémité des moyens d'étanchéité (20) se situe dans une rainure (26) dans l'élément de transport (4) pour former un joint avec celui-ci quand la région renfoncée (12) de l'élément de transport (4) est en correspondance avec la pile de capteurs (16).

4. Dispositif d'analyse selon la revendication 3, dans lequel les moyens d'étanchéité comprennent une gaine rétractable (20) qui s'accouple de façon étanche dans la rainure (26) de l'élément de transport (4) quand elle se trouve dans une configuration sortie et qui ne forme pas de joint avec l'élément de transport (4) quand elle se trouve dans une configuration rentrée.

5. Dispositif d'analyse selon l'une quelconque des revendications précédentes, dans lequel un poussoir (28) est prévu pour conférer un mouvement de translation à un capteur (16) installé dans ladite région renfoncée (12) pendant et/ou après la rotation de l'élément de transport (4) de façon à amener le capteur (16) jusqu'à l'emplacement d'accouplement.

6. Dispositif d'analyse selon la revendication 5, dans lequel le poussoir (28) est installé sur l'élément de transport (4) et une partie du poussoir (28) est située sur une piste hélicoïdale (30) dans le boîtier (2), de manière à ce que la rotation de l'élément de transport (4) confère un mouvement de translation au poussoir (28).

7. Dispositif d'analyse selon la revendication 1, dans lequel ladite ouverture est l'unique ouverture vers l'intérieur du boîtier (2), et dans lequel les moyens d'étanchéité comprennent un joint (20) qui est fixé par rapport à une surface extérieure de l'élément de transport (4) et qui obture l'ouverture du boîtier (2) lorsque l'élément de transport (4) se trouve dans une position de rotation spécifiée.

8. Dispositif d'analyse selon la revendication 2, dans lequel les moyens d'étanchéité comprennent un joint (20) qui est fixé par rapport à une surface extérieure de l'élément de transport (4) et qui obture l'ouverture du chargeur (18) lorsque l'élément de transport (4) se trouve dans une position de rotation spécifiée.

9. Dispositif d'analyse selon la revendication 1, dans lequel ladite ouverture est l'unique ouverture vers l'intérieur du boîtier (2), et dans lequel les moyens d'étanchéité comprennent un joint (66) prévu sur une porte (52) qui est adaptée pour s'ajuster à ladite ouverture de sorte que le joint étanche à l'humidité est effectué par la fermeture de la porte (52) ; dans lequel la porte (52) est raccordée de façon opérationnelle à l'élément de transport (4) de sorte que la porte (52) sera ouverte lorsque l'élément de transport (4) se trouve dans une première position de rotation et fermée lorsque l'élément de transport (4) se trouve dans une deuxième position de rotation.

10. Dispositif d'analyse selon la revendication 2, dans lequel les moyens d'étanchéité comprennent un joint (66) prévu sur une porte (52) qui est adaptée pour s'ajuster à l'ouverture du chargeur (18) de sorte que le joint étanche à l'humidité est effectué par la fermeture de la porte (52) ; dans lequel la porte (52) est raccordée de façon opérationnelle à l'élément de transport (4) de sorte que la porte (52) sera ouverte lorsque l'élément de transport (4) se trouve dans une première position de rotation et fermée lorsque l'élément de transport (4) se trouve dans une deuxième position de rotation.

11. Dispositif d'analyse selon la revendication 9 ou 10, dans lequel la porte (52) est munie d'une ou de plusieurs dents (54) qui restreignent le mouvement de la pile de capteurs (16) contre la force des moyens de ressort (24).

12. Dispositif d'analyse selon la revendication 11, dans lequel l'élément de transport (4) est muni d'au moins une lame (62) qui prend en charge la fonction de restriction de la pile de capteurs (16) quand la porte (52) est ouverte.

13. Dispositif d'analyse selon l'une quelconque des revendications 9 à 12, dans lequel la porte (52) est installée de façon pivotante par rapport au boîtier (2).

14. Dispositif d'analyse selon l'une quelconque des revendications précédentes, dans lequel l'élément de transport (4) est raccordé de façon opérationnelle à un ressort de rappel (38) qui pousse l'élément de transport (4) à adopter une position de rotation spécifiée à laquelle les moyens d'étanchéité (20, 66) peuvent fournir un joint étanche à l'humidité entre la pile de capteurs (16) et l'élément de transport (4).

15. Dispositif d'analyse selon l'une quelconque des revendications précédentes, dans lequel une partie du capteur (16) à laquelle un échantillon fluide doit être appliqué n'est pas supportée par l'élément de transport (4) lorsqu'elle se trouve à l'emplacement d'accouplement.

16. Dispositif d'analyse selon l'une quelconque des revendications précédentes, dans lequel l'élément de transport (4) présente un profil externe qui est de section transversale substantiellement circulaire.

17. Dispositif d'analyse selon l'une quelconque des revendications précédentes, dans lequel chaque capteur (16) dans la pile comprend un élément de base présentant une zone opérationnelle à laquelle le fluide est appliqué, contenant les moyens réactifs, et une zone non opérationnelle (44) adjacente à la zone opérationnelle, dans lequel l'épaisseur totale du capteur (16) dans au moins une partie de la zone non opérationnelle (44) est au moins aussi grande que l'épaisseur totale du capteur (16) dans la zone opérationnelle.

18. Dispositif d'analyse selon la revendication 17, dans lequel l'épaisseur totale du capteur (16) dans au moins une partie de la zone non opérationnelle (44) est supérieure à l'épaisseur totale du capteur (16) dans la zone opérationnelle.

19. Dispositif d'analyse selon l'une quelconque des revendications précédentes, comprenant en outre des moyens de mise en charge (70) permettant d'appliquer une charge de compression à un capteur (16) pendant au moins une partie du temps lorsque ledit capteur (16) se trouve dans la région renfoncée (12) de l'élément de transport (4).

20. Dispositif d'analyse selon l'une quelconque des revendications précédentes, comprenant en outre des moyens antiretour (78) qui empêchent ou inhibent le transport d'un capteur (16) de l'emplacement d'accouplement au chargeur (18) et qui empêchent ou inhibent la réintroduction d'un capteur usagé éjecté (16) dans l'emplacement d'accouplement.

21. Dispositif d'analyse selon la revendication 20, dans lequel lesdits moyens antiretour (78) et lesdits moyens de mise en charge (70) comprennent un seul composant élastique et souple.

22. Dispositif d'analyse selon l'une quelconque des revendications précédentes, comprenant en outre des moyens de cliquet (82, 84) associés à la pile de capteurs (16) qui empêchent ou inhibent le mouvement de la pile dans une direction opposée à celle dans laquelle les moyens de ressort (24) poussent la pile.

23. Dispositif d'analyse selon l'une quelconque des revendications précédentes, adapté pour une utilisation dans l'analyse de la concentration en glucose dans le sang.
